(19) Europäisches Patentamt
European Patent Office
Office européen des brevets

(11) EP 4 084 012 A1

(12) **EUROPEAN PATENT APPLICATION**
published in accordance with Art. 153(4) EPC

(43) Date of publication:
02.11.2022 Bulletin 2022/44

(21) Application number: 20905202.6

(22) Date of filing: 23.12.2020

(51) International Patent Classification (IPC):
*G16H 50/20* (2018.01)

(52) Cooperative Patent Classification (CPC):
G16H 50/20

(86) International application number:
PCT/JP2020/048146

(87) International publication number:
WO 2021/132323 (01.07.2021 Gazette 2021/26)

(84) Designated Contracting States:
AL AT BE BG CH CY CZ DE DK EE ES FI FR GB
GR HR HU IE IS IT LI LT LU LV MC MK MT NL NO
PL PT RO RS SE SI SK SM TR
Designated Extension States:
BA ME
Designated Validation States:
KH MA MD TN

(30) Priority: 24.12.2019 JP 2019232495

(71) Applicant: E-Solutions, Inc.
Tokyo 105-6017 (JP)

(72) Inventor: SASAKI, Keishin
Tokyo 105-6017 (JP)

(74) Representative: Pitchford, James Edward et al
Mathys & Squire
The Shard
32 London Bridge Street
London SE1 9SG (GB)

(54) **HEALTH MANAGEMENT SYSTEM AND HEALTH MANAGEMENT METHOD**

(57) A health management system includes a risk calculator 301 for receiving input of vital data on a human or an animal, and for calculating a disease occurrence risk in the human or the animal; and a reduction measure specifier 302 for specifying a reduction measure for reducing the disease occurrence risk in the human or the animal.

Fig. 1

**Description**

Technical Field

The present invention relates to a health management system and a health management method.

Background Art

Proposals have been made to measure vital data on a human or an animal (for example, see Patent Literatures 1 to 6).

Citation List

Patent Literature

Patent Literature 1: Japanese Patent No. 6203765
Patent Literature 2: Japanese Patent No. 6217787
Patent Literature 3: Japanese Patent No. 6288485
Patent Literature 4: Japanese Patent No. 6342095
Patent Literature 5: Japanese Patent No. 6566241
Patent Literature 6: Japanese Patent No. 6577761

Summary of Invention

Technical Problem

An object of the present invention is to provide a health management system and a health management method capable of improving a health state of a human or an animal.

Solution to Problem

According to an aspect of the present invention, there is provided a health management system including:

a risk calculator for receiving input of vital data on a human or an animal, and for calculating a disease occurrence risk in the human or the animal; and
a reduction measure specifier for specifying a reduction measure for reducing the disease occurrence risk in the human or the animal.

The health management system described above may further include a measure for measuring the vital data.
With the health management system described above, the measure may be disposed in a building where the human or the animal is present.
With the health management system described above, the measure may be attached to the human or the animal.
The health management system described above may further include an input device for receiving input of the vital data from the human, and for transmitting the vital data to the risk calculator.
With the health management system described above, the risk calculator may receive input of the vital data on the human or the animal from a medical facility.
The health management system described above may further include a reduction measure information transmitter for transmitting information about the reduction measure that is specified.
The health management system described above may further include a disease occurrence risk database for storing a correlation between the vital data and the disease occurrence risk, where
the risk calculator may calculate the disease occurrence risk by accessing the disease occurrence risk database.
The health management system described above may further include a reduction measure database for storing a correlation between the disease occurrence risk and the reduction measure.
With the health management system described above, the risk calculator may receive input of the vital data on the human or the animal subjected to the reduction measure, and may calculate the disease occurrence risk in the human or the animal subjected to the reduction measure.

The health management system described above may further include a comparator for comparing the disease occurrence risk in the human or the animal before application of the reduction measure, and the disease occurrence risk in the human or the animal after application of the reduction measure.

The health management system described above may further include a reduction measure database for storing a correlation between the disease occurrence risk and the reduction measure, where

the reduction measure specifier may specify the reduction measure by accessing the reduction measure database, and

the health management system may further include a correlation evaluator for increasing a degree of reliability of the correlation between the disease occurrence risk and the reduction measure and/or strength of the correlation in a case where the disease occurrence risk in the human or the animal after application of the reduction measure is reduced compared to the disease occurrence risk in the human or the animal before application of the reduction measure.

With the health management system described above, in a case where the disease occurrence risk in the human or the animal after application of the reduction measure is increased compared to the disease occurrence risk in the human or the animal before application of the reduction measure, the correlation evaluator may reduce the degree of reliability of the correlation between the disease occurrence risk and the reduction measure and/or the strength of the correlation.

With the health management system described above, the risk calculator may receive input of the vital data on the human or the animal not subjected to the reduction measure and the vital data on the human or the animal subjected to the reduction measure, and may calculate the disease occurrence risk in the human or the animal not subjected to the reduction measure and the disease occurrence risk in the human or the animal subjected to the reduction measure.

The health management system described above may further include a comparator for comparing the disease occurrence risk in the human or the animal not subjected to the reduction measure, and the disease occurrence risk in the human or the animal subjected to the reduction measure.

The health management system described above may further include a reduction measure database for storing a correlation between the disease occurrence risk and the reduction measure, where

the reduction measure specifier may specify the reduction measure by accessing the reduction measure database, and

the health management system may further include a correlation evaluator for increasing a degree of reliability of the correlation between the disease occurrence risk and the reduction measure and/or strength of the correlation in a case where the disease occurrence risk in the human or the animal subjected to the reduction measure is reduced compared to the disease occurrence risk in the human or the animal not subjected to the reduction measure.

With the health management system described above, in a case where the disease occurrence risk in the human or the animal subjected to the reduction measure is increased compared to the disease occurrence risk in the human or the animal not subjected to the reduction measure, the correlation evaluator may reduce the degree of reliability of the correlation between the disease occurrence risk and the reduction measure and/or the strength of the correlation.

The health management system described above may further include a product/service specifier for specifying a product or a service for implementing the reduction measure.

The health management system described above may further include a product/service information transmitter for transmitting information about the product or the service that is specified.

The health management system described above may further include a product/service database for storing a correlation between the reduction measure and the product or the service, where the product/service specifier may specify the product or the service by accessing the product/service database.

With the health management system described above, the risk calculator may receive input of the vital data on the human or the animal that used the product or the service, and may calculate the disease occurrence risk in the human or the animal that used the product or the service.

The health management system described above may further include a comparator for comparing the disease occurrence risk in the human or the animal before use of the product or the service, and the disease occurrence risk in the human or the animal after use of the product or the service.

The health management system described above may further include a product/service database for storing a correlation between the reduction measure and the product or the service, where

the product/service specifier may specify the product or the service by accessing the product/service database, and

a degree of reliability of the correlation between the reduction measure and the product or the service and/or strength of the correlation may be increased in a case where the disease occurrence risk in the human or the animal after use of the product or the service is reduced compared to the disease occurrence risk in the human or the animal before use of the product or the service.

The health management system described above may further include a correlation evaluation result transmitter for transmitting a notification indicating that the degree of reliability of the correlation and/or the strength of the correlation is increased, to a provider of the product or the service.

With the health management system described above, in a case where the disease occurrence risk in the human or the animal after use of the product or the service is increased compared to the disease occurrence risk in the human or the animal before use of the product or the service, the correlation evaluator may reduce the degree of reliability of the correlation between the reduction measure and the product or the service and/or the strength of the correlation.

The health management system described above may further include a correlation evaluation result transmitter for transmitting a notification indicating that the degree of reliability of the correlation and/or the strength of the correlation is reduced, to a provider of the product or the service.

With the health management system described above, the risk calculator may receive input of the vital data on the human or the animal that did not use the product or the service and the vital data on the human or the animal that used the product or the service, and may calculate the disease occurrence risk in the human or the animal that did not use the product or the service and the disease occurrence risk in the human or the animal that used the product or the service.

The health management system described above may further include a comparator for comparing the disease occurrence risk in the human or the animal that did not use the product or the service, and the disease occurrence risk in the human or the animal that used the product or the service.

The health management system described above may further include a product/service database for storing a correlation between the reduction measure and the product or the service, where

the product/service specifier may specify the product or the service by accessing the product/service database, and the health management system may further include a correlation evaluator for increasing a degree of reliability of the correlation between the reduction measure and the product or the service and/or strength of the correlation in a case where the disease occurrence risk in the human or the animal that used the product or the service is reduced compared to the disease occurrence risk in the human or the animal that did not use the product or the service.

The health management system described above may further include a correlation evaluation result transmitter for transmitting a notification indicating that the degree of reliability of the correlation and/or the strength of the correlation is increased, to a provider of the product or the service.

With the health management system described above, in a case where the disease occurrence risk in the human or the animal that used the product or the service is increased compared to the disease occurrence risk in the human or the animal that did not use the product or the service, the correlation evaluator may reduce the degree of reliability of the correlation between the reduction measure and the product or the service and/or the strength of the correlation.

The health management system described above may further include a correlation evaluation result transmitter for transmitting a notification indicating that the degree of reliability of the correlation and/or the strength of the correlation is reduced, to a provider of the product or the service.

With the health management system described above, the vital data may include at least one of sex, age, height, and weight of the human or the animal.

With the health management system described above, the vital data may include at least one of a past medical history and a family medical history of the human or the animal.

With the health management system described above, the vital data may include at least one of a heart rate, respiration, a body movement, a body temperature, an ambient temperature, and an ambient humidity of the human or the animal.

With the health management system described above, the vital data may include at least one of blood pressure, a biochemical test result, and a hematology test result of the human or the animal.

With the health management system described above, the vital data may include an ingested substance of the human or the animal.

With the health management system described above, the vital data may include a medical treatment received by the human or the animal, and a medication administration history.

With the health management system described above, the vital data may include genetic information on the human or the animal.

Furthermore, according to an aspect of the present invention, there is provided a health management method including:

receiving input of vital data on a human or an animal, and calculating a disease occurrence risk in the human or the animal, the receiving and the calculating being performed by a risk calculator of a computer system; and
specifying, by a reduction measure specifier of the computer system, a reduction measure for reducing the disease occurrence risk in the human or the animal.

The health management method described above may further include measuring the vital data by a measure.
In the health management method described above, the measure may be disposed in a building where the human or the animal is present.
In the health management method described above, the measure may be attached to the human or the animal.
The health management method described above may further include receiving input of the vital data from the human, and transmitting the vital data to the risk calculator, the receiving and the transmitting being performed by an input device.
The health management method described above may further include receiving, by the risk calculator, input of the vital data on the human or the animal from a medical facility.
The health management method described above may further include transmitting, by a reduction measure information transmitter of the computer system, information about the reduction measure that is specified.
The health management method described above may further include preparing a disease occurrence risk database for storing a correlation between the vital data and the disease occurrence risk, where
the risk calculator may calculate the disease occurrence risk by accessing the disease occurrence risk database.
The health management method described above may further include preparing a reduction measure database for storing a correlation between the disease occurrence risk and the reduction measure, where
the reduction measure specifier may specify the reduction measure by accessing the reduction measure database.
The health management method described above may further include receiving input of the vital data on the human or the animal subjected to the reduction measure, and calculating the disease occurrence risk in the human or the animal subjected to the reduction measure, the receiving and the calculating being performed by the risk calculator.
The health management method described above may further include comparing, by a comparator of the computer system, the disease occurrence risk in the human or the animal before application of the reduction measure, and the disease occurrence risk in the human or the animal after application of the reduction measure.
The health management method described above may further include

preparing a reduction measure database for storing a correlation between the disease occurrence risk and the reduction measure;
specifying, by the reduction measure specifier, the reduction measure by accessing the reduction measure database; and
increasing, by a correlation evaluator of the computer system, a degree of reliability of the correlation between the disease occurrence risk and the reduction measure and/or strength of the correlation in a case where the disease occurrence risk in the human or the animal after application of the reduction measure is reduced compared to the disease occurrence risk in the human or the animal before application of the reduction measure.

The health management method described above may further include reducing, by the correlation evaluator, the degree of reliability of the correlation between the disease occurrence risk and the reduction measure and/or the strength of the correlation in a case where the disease occurrence risk in the human or the animal after application of the reduction measure is increased compared to the disease occurrence risk in the human or the animal before application of the reduction measure.
The health management method described above may further include receiving input of the vital data on the human or the animal not subjected to the reduction measure and the vital data on the human or the animal subjected to the reduction measure, and calculating the disease occurrence risk in the human or the animal not subjected to the reduction measure and the disease occurrence risk in the human or the animal subjected to the reduction measure, the receiving and the calculating being performed by the risk calculator.
The health management method described above may further include comparing, by a comparator of the computer system, the disease occurrence risk in the human or the animal not subjected to the reduction measure, and the disease occurrence risk in the human or the animal subjected to the reduction measure.
The health management method described above may further include:

preparing a reduction measure database for storing a correlation between the disease occurrence risk and the reduction measure;

specifying, by the reduction measure specifier, the reduction measure by accessing the reduction measure database; and

increasing, by a correlation evaluator of the computer system, a degree of reliability of the correlation between the disease occurrence risk and the reduction measure and/or strength of the correlation in a case where the disease occurrence risk in the human or the animal subjected to the reduction measure is reduced compared to the disease occurrence risk in the human or the animal not subjected to the reduction measure.

The health management method described above may further include reducing, by the correlation evaluator, the degree of reliability of the correlation between the disease occurrence risk and the reduction measure and/or the strength of the correlation in a case where the disease occurrence risk in the human or the animal subjected to the reduction measure is increased compared to the disease occurrence risk in the human or the animal not subjected to the reduction measure.

The health management method described above may further include specifying, by a product/service specifier of the computer system, a product or a service for implementing the reduction measure.

The health management method described above may further include transmitting, by a product/service information transmitter of the computer system, information about the product or the service that is specified.

The health management method described above may further include preparing a product/service database for storing a correlation between the reduction measure and the product or the service, where

the product/service specifier may specify the product or the service by accessing the product/service database.

The health management method described above may further include receiving input of the vital data on the human or the animal that used the product or the service, and calculating the disease occurrence risk in the human or the animal that used the product or the service, the receiving and the calculating being performed by the risk calculator.

The health management method described above may further include comparing, by a comparator of the computer system, the disease occurrence risk in the human or the animal before use of the product or the service, and the disease occurrence risk in the human or the animal after use of the product or the service.

The health management method described above may further include:

preparing a product/service database for storing a correlation between the reduction measure and the product or the service;

specifying, by the product/service specifier, the product or the service by accessing the product/service database; and

increasing, by a correlation evaluator of the computer system, a degree of reliability of the correlation between the reduction measure and the product or the service and/or strength of the correlation in a case where the disease occurrence risk in the human or the animal after use of the product or the service is reduced compared to the disease occurrence risk in the human or the animal before use of the product or the service.

The health management method described above may further include transmitting, by a correlation evaluation result transmitter of the computer system, a notification indicating that the degree of reliability of the correlation and/or the strength of the correlation is increased, to a provider of the product or the service.

The health management method described above may further include reducing, by the correlation evaluator, the degree of reliability of the correlation between the reduction measure and the product or the service and/or the strength of the correlation in a case where the disease occurrence risk in the human or the animal after use of the product or the service is increased compared to the disease occurrence risk in the human or the animal before use of the product or the service.

The health management method described above may further include transmitting, by a correlation evaluation result transmitter of the computer system, a notification indicating that the degree of reliability of the correlation and/or the strength of the correlation is reduced, to a provider of the product or the service.

The health management method described above may further include receiving input of the vital data on the human or the animal that did not use the product or the service and the vital data on the human or the animal that used the product or the service, and calculating the disease occurrence risk in the human or the animal that did not use the product or the service and the disease occurrence risk in the human or the animal that used the product or the service, the receiving and the calculating being performed by the risk calculator.

The health management method described above may further include comparing, by a comparator of the computer system, the disease occurrence risk in the human or the animal that did not use the product or the service, and the disease occurrence risk in the human or the animal that used the product or the service.

The health management method described above may further include:

preparing a product/service database for storing a correlation between the reduction measure and the product or the service;

specifying, by the product/service specifier, the product or the service by accessing the product/service database; and increasing, by a correlation evaluator of the computer system, a degree of reliability of the correlation between the reduction measure and the product or the service and/or strength of the correlation in a case where the disease occurrence risk in the human or the animal that used the product or the service is reduced compared to the disease occurrence risk in the human or the animal that did not use the product or the service.

The health management method described above may further include transmitting, by a correlation evaluation result transmitter of the computer system, a notification indicating that the degree of reliability of the correlation and/or the strength of the correlation is increased, to a provider of the product or the service.

The health management method described above may further include reducing, by the correlation evaluator, the degree of reliability of the correlation between the reduction measure and the product or the service and/or the strength of the correlation in a case where the disease occurrence risk in the human or the animal that used the product or the service is increased compared to the disease occurrence risk in the human or the animal that did not use the product or the service.

The health management method described above may further include transmitting, by a correlation evaluation result transmitter of the computer system, a notification indicating that the degree of reliability of the correlation and/or the strength of the correlation is reduced, to a provider of the product or the service.

In the health management method described above, the vital data may include at least one of sex, age, height, and weight of the human or the animal.

In the health management method described above, the vital data may include at least one of a past medical history and a family medical history of the human or the animal.

In the health management method described above, the vital data may include at least one of a heart rate, respiration, a body movement, a body temperature, an ambient temperature, and an ambient humidity of the human or the animal.

In the health management method described above, the vital data may include at least one of blood pressure, a biochemical test result, and a hematology test result of the human or the animal.

In the health management method described above, the vital data may include an ingested substance of the human or the animal.

In the health management method described above, the vital data may include a medical treatment received by the human or the animal, and a medication administration history.

In the health management method described above, the vital data may include genetic information on the human or the animal.

Advantageous Effect of Invention

According to the present invention, a health management system and a health management method capable of improving a health state of a human or an animal may be provided.

Brief Description of Drawings

[Figure 1] Figure 1 is a schematic diagram showing a health management system according to an embodiment.
[Figure 2] Figure 2 is a schematic diagram showing the health management system according to the embodiment.
[Figure 3] Figure 3 is a schematic diagram showing the health management system according to the embodiment.
[Figure 4] Figure 4 is a schematic diagram showing the health management system according to the embodiment.
[Figure 5] Figure 5 is a flowchart showing a health management method according to the embodiment.
[Figure 6] Figure 6 is a flowchart showing the health management method according to the embodiment.
[Figure 7] Figure 7 is a flowchart showing the health management method according to the embodiment.
[Figure 8] Figure 8 is a flowchart showing the health management method according to the embodiment.
[Figure 9] Figure 9 is a flowchart showing the health management method according to the embodiment.

Description of Embodiment

Hereinafter, an embodiment of the present invention will be described. In the following description of the drawings, same or similar parts are denoted by same or similar reference signs. However, the drawings are schematic. Accordingly, specific dimensions and the like should be determined in light of the description given below. Moreover, the relationship or ratio of dimensions may of course differ among drawings.

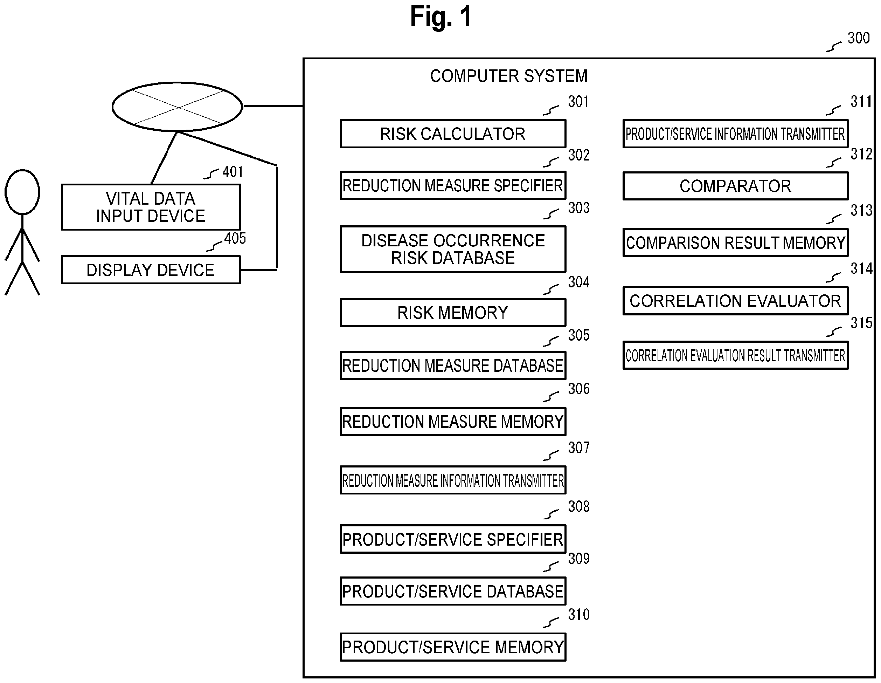

As shown in Figure 1, a health management system according to the embodiment includes a risk calculator 301 for receiving input of vital data on a human or an animal, and for calculating a disease occurrence risk in the human or the animal, and a reduction measure specifier 302 for specifying a reduction measure for reducing the disease occurrence risk in the human or the animal. The risk calculator 301 and the reduction measure specifier 302 are included in a computer system 300, for example.

The vital data is any data regarding life of a human or an animal, and is not limited to specific data. Examples of the vital data include sex, age, height, and weight of the human or the animal, and whether the human or the animal is pregnant. Examples of the vital data further include a past medical history and a family medical history of the human or the animal. The past medical history is a medical history of the human or the animal, and the family medical history is a medical history of the family of the human or the animal that is a management target. The family medical history is a medical history of parents, siblings, children, and other relatives of the human or the animal, for example. In the case where the human or the animal that is the management target includes a plurality of humans or a plurality of animals that are blood relatives, the past medical history of one of them may be automatically acquired as the family medical history of another one that is a blood relative. Moreover, examples of the vital data may include a medical treatment received by the human or the animal, and a medication administration history.

Examples of the vital data may further include a heart rate, respiration, a body movement, a body temperature, an ambient temperature, and an ambient humidity of the human or the animal. Examples of the body movement may include the number of steps, a movement distance, and a movement speed. The body movement may be motion of a part of the body of the human or the animal. Furthermore, examples of the vital data may include a blood pressure, a biochemical test result, and a hematology test result of the human or the animal.

Examples of the biochemical test result may include the total amount of protein including albumin and globulin, the amount of albumin, the albumin-globulin ratio, the total amount of bilirubin, the amount of direct bilirubin, the amount of AST, the amount of ALT, the amount of LDH, the amount of alkaline phosphatase, the amount of $\gamma$-GTP, the amount of cholinesterase, the amount of CPK, the amount of amylase, the amount of glucose (blood glucose level), the amount of HbA1c, the amount of sodium, the amount of potassium, the amount of chlorine, the amount of CRP, the amount of triglycerides, the total amount of cholesterol, the amount of HDL-C, the amount of urea nitrogen, the amount of creatinine, the amount of uric acid, and the amount of iron.

Examples of the hematology test result may include the white blood cell count, the red blood cell count, the hemoglobin level, the hematocrit level, the platelet count, the neutrophil count, the lymphocyte count, the monocyte count, the eosinophil count, the basophil count, the prothrombin time, the active partial thromboplastin time, the fibrinogen level, and the D-dimer level.

Furthermore, examples of the vital data may include an ingested substance of the human or the animal. Examples of the ingested substance may include food and beverages such as milk beverage, tea, coffee, juice and alcoholic beverage that are ingested at breakfast, lunch, and dinner and as between-meal snacks. Moreover, the vital data may further include data about a sugar content, a salt content, a fat content, a calorie content, and the like of the ingested substance.

Furthermore, as an example of the vital data, genetic information on the human or the animal may be cited.

Means for inputting the vital data to the risk calculator 301 is not particularly specified. For example, a vital data input device 401 for inputting the vital data may be connected to the risk calculator 301. For example, the vital data input device 401 receives input of the vital data from the human, and transmits the same to the risk calculator. Examples of the vital data input device 401 may include a keyboard, a mouse, a personal computer, and a mobile terminal. The vital data input device 401 may be connected to the risk calculator 301 in a wired or wireless manner. The vital data input device 401 may be connected to the risk calculator 301 over a network. The vital data input device 401 may be disposed near the risk calculator 301 or may be disposed at a remote location.

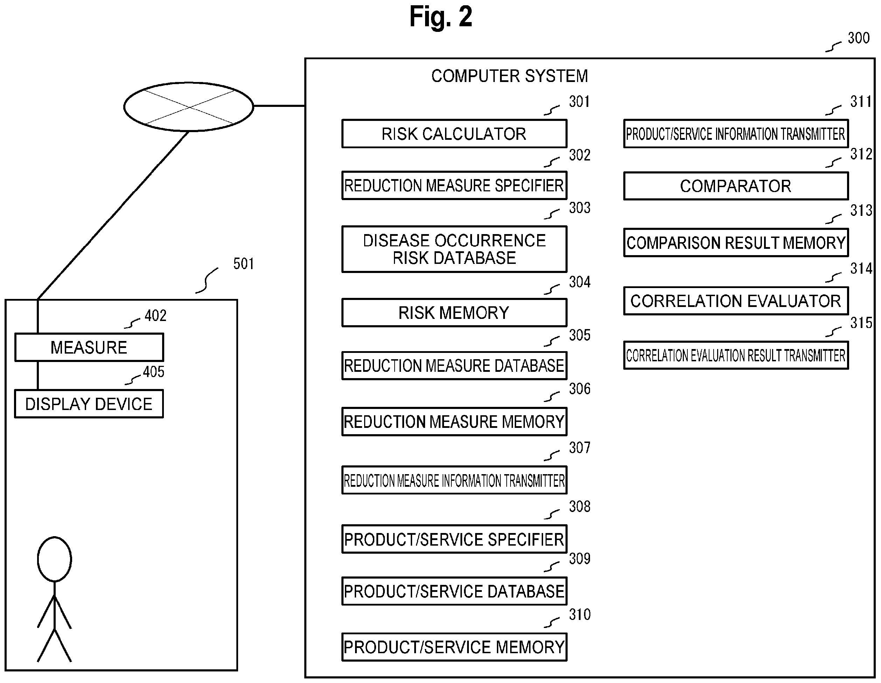

Furthermore, as shown in Figure 2, a measure 402 that is a device for measuring the vital data on the human or the animal may be connected to the risk calculator 301, for example. Examples of the measure 402 may include a height meter, a weight scale, a heart rate meter, a respirometer, a body movement sensor, a pedometer, a movement distance sensor, a movement speed sensor, a blood pressure gauge, a biochemical testing device, a hematology testing device, and a genetic testing device. The measure 402 may be disposed in a building 501 where the human or the animal is present. Examples of the building 501 may include residential buildings such as a house and an apartment, and commercial facilities and community facilities such as an accommodation facility, a boarding house, a shop, a concert hall, an art museum, a museum, an aquarium, a hospital, an office, and a factory. The measure 402 may be disposed in a room in the building 501, such as a living room or a bedroom. The measure 402 may be attached to the human or the animal. The measure 402 may be a wearable terminal. The measure 402 may be connected to the risk calculator 301 in a wired or wireless manner. The measure 402 may be connected to the risk calculator 301 over a network. The measure 402 may be disposed near the risk calculator 301 or may be disposed at a remote location.

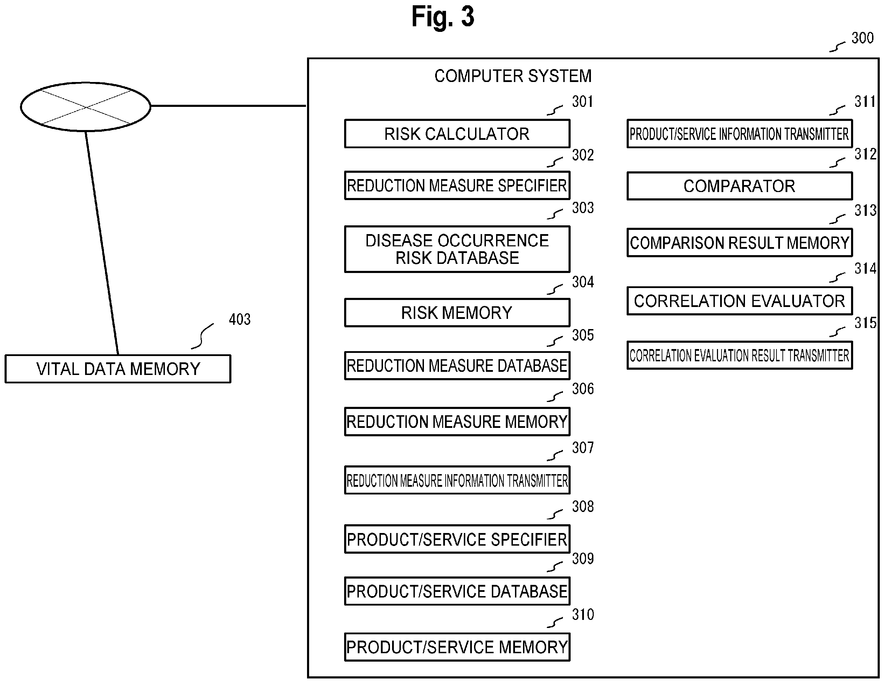

Furthermore, as shown in Figure 3, a vital data memory 403 for storing the vital data on the human or the animal may be connected to the risk calculator 301, for example. The vital data memory 403 stores the vital data on the human or the animal that is measured in advance or that is input. The risk calculator 301 may receive input of the vital data on the human or the animal by reading out the vital data on the human or the animal from the vital data memory 403. The vital data memory 403 may be connected to the risk calculator 301 in a wired or wireless manner. The vital data memory 403 may be connected to the risk calculator 301 over a network. The vital data memory 403 may be disposed near the risk calculator 301 or may be disposed at a remote location.

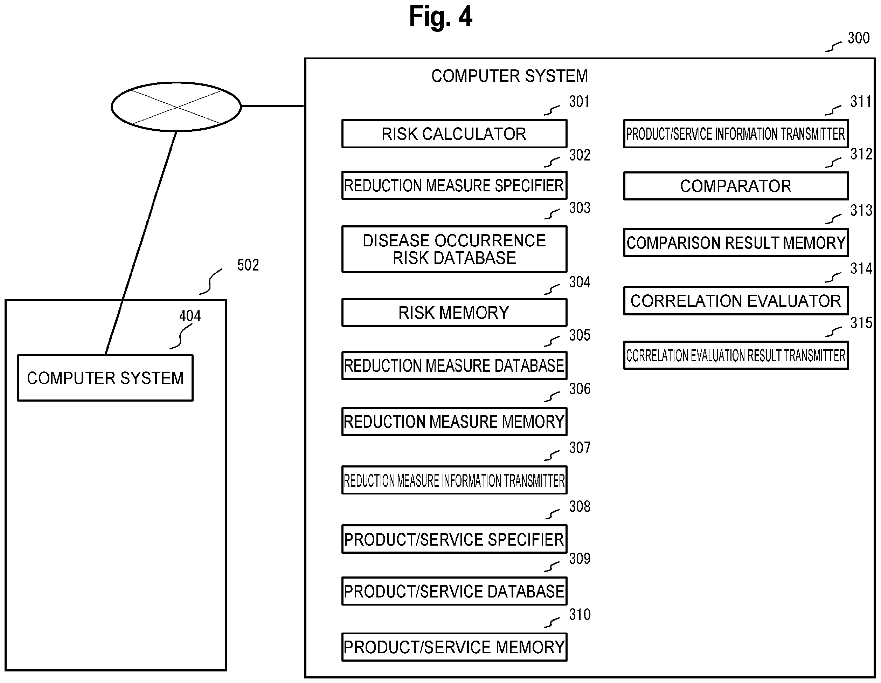

Furthermore, as shown in Figure 4, the risk calculator 301 may receive input of the vital data on the human or the animal from a medical facility 502, for example. Examples of the medical facility 502 may include a hospital, a clinic, a health center, an animal hospital, and a research center. For example, a computer system 404 in the medical facility 502 transmits the vital data on the human or the animal collected at the medical facility 502 to the risk calculator 301. The computer system 404 in the medical facility 502 may be connected to the risk calculator 301 in a wired or wireless manner. The computer system 404 in the medical facility 502 may be connected to the risk calculator 301 over a network. The computer system 404 in the medical facility 502 may be disposed near the risk calculator 301 or may be disposed at a remote location.

There may be more than one means for inputting the vital data to the risk calculator 301. For example, one or more means may be selected from a combination of the vital data input device 401 shown in Figure 1, the measure 402 shown in Figure 2, the vital data memory 403 shown in Figure 3, and the computer system 404 in the medical facility 502 shown in Figure 4.

A disease that is a target of calculation of an occurrence risk by the risk calculator 301 shown in Figures 1 to 4 is not particularly specified. Examples of the disease may include cancer, diabetes, ischemic heart disease, dementia, hypertension, arrhythmia, cerebral apoplexy, and cardiac arrest. The risk calculator 301 calculates the disease occurrence risk in the human or the animal based on the vital data on the human or the animal that is input. For example, the risk calculator 301 calculates the ratio of the disease occurrence risk in the human or the animal that is the management target to that of a human or an animal whose vital data is within a normal range. For example, the risk calculator 301 calculates the disease occurrence risk according to the vital data on the human or the animal that is the management target. For example, in the case where the ingested amount of sugar or the blood glucose level of the human or the animal that is the management target is high, the risk calculator 301 calculates the occurrence risk of diabetes according to the vital data.

The health management system according to the embodiment may further include a disease occurrence risk database 303 for storing a correlation between the vital data and the disease occurrence risk. The disease occurrence risk database 303 is included in the computer system, for example. The risk calculator 301 may access the disease occurrence risk database 303, and may calculate the disease occurrence risk based on the vital data on the human or the animal that is input, and the correlation between the vital data and the disease occurrence risk.

The correlation between the vital data and the disease occurrence risk is not particularly specified. For example, the disease occurrence risk database 303 stores an algorithm defining the correlation between the vital data and the disease occurrence risk. The correlation between the vital data and the disease occurrence risk is given by Equation (1) below, for example.

$$y = f(x_1, x_2, x_3, ..., x_n) \quad (1)$$

In Equation (1), n is a natural number, $x_n$ indicates n independent variables in the vital data, and y indicates a dependent variable of the disease occurrence risk. The number of types of vital data may be any number. The disease occurrence risk database 303 stores the correlation between the vital data and the disease occurrence risk for each disease. The correlation between the vital data and the disease occurrence risk is acquired in advance. For example, the correlation between the vital data and the disease occurrence risk may be acquired in advance and stored in the disease occurrence risk database 303 by statistically analyzing the vital data on a large number of subjects and actual disease occurrence in the subjects.

The health management system according to the embodiment may further include a risk memory 304 for storing the disease occurrence risk in the human or the animal that is calculated by the risk calculator 301. For example, the risk memory 304 is included in the computer system. The risk calculator 301 stores the disease occurrence risk in the human or the animal that is calculated, in the risk memory 304.

The reduction measure specifier 302 reads out the disease occurrence risk in the human or the animal that is the management target, from the risk memory 304. Furthermore, the reduction measure specifier 302 specifies the reduction measure for reducing the aforementioned disease occurrence risk. The reduction measure is not limited to a specific measure as long as the measure is for reducing the disease occurrence risk in the human or the animal.

The health management system according to the embodiment may further include a reduction measure database 305 for storing a correlation between the disease occurrence risk and the reduction measure. For example, the reduction measure database 305 is included in the computer system. The correlation between the disease occurrence risk and the reduction measure is not particularly specified. For example, in relation to an occurrence risk of ischemic heart disease, smoking cessation and exercise are registered in the reduction measure database 305 as the reduction measures. The reduction measure database 305 may also store a degree of reliability and/or strength of the correlation between the disease occurrence risk and the reduction measure.

For example, the reduction measure specifier 302 accesses the reduction measure database 305, and specifies the reduction measure for reducing the disease occurrence risk in the human or the animal that is the management target, based on the disease occurrence risk in the human or the animal that is the management target and the correlation between the disease occurrence risk and the reduction measure. In the case where a plurality of reduction measures are registered in the reduction measure database 305 in relation to the disease occurrence risk, the reduction measure specifier 302 may extract a reduction measure with a high degree of reliability and/or high strength of correlation to the disease occurrence risk, by excluding a reduction measure with a low degree of reliability and/or low strength of correlation to the disease occurrence risk.

The health management system according to the embodiment may further include a reduction measure memory 306 for storing the reduction measure, specified by the reduction measure specifier 302, for reducing the disease occurrence risk in the human or the animal that is the management target. For example, the reduction measure memory 306 is included in the computer system. The reduction measure specifier 302 stores the specified reduction measure for reducing the disease occurrence risk in the human or the animal that is the management target, in the reduction measure memory 306.

The health management system according to the embodiment may further include a reduction measure information transmitter 307 for transmitting information about the reduction measure that is specified by the reduction measure specifier 302. For example, the reduction measure information transmitter 307 is included in the computer system. For example, the reduction measure information transmitter 307 reads out the reduction measure for reducing the disease occurrence risk in the human or the animal that is the management target, from the reduction measure memory 306, and transmits information about the reduction measure to a manager or the like of the management target human or the management target animal. For example, the reduction measure information transmitter 307 transmits the information about the reduction measure to a display device 405 that is capable of displaying the information about the reduction measure to the manager or the like of the management target human or the management target animal. For example, the display device 405 may be included in a television, a personal computer, a mobile terminal or the like. The display device 405 may be connected to the reduction measure information transmitter 307 in a wired or wireless manner. The display device 405 may be connected to the reduction measure information transmitter 307 over a network. The display device 405 may be disposed near the reduction measure information transmitter 307 or may be disposed at a remote location.

The health management system according to the embodiment may further include a product/service specifier 308 for specifying a product or a service for implementing the reduction measure specified by the reduction measure specifier 302. For example, the product/service specifier 308 is included in the computer system. The product or the service may be any product or service as long as the product or the service is for implementing the reduction measure for reducing the disease occurrence risk in the human or the animal. The product/service specifier 308 reads out the reduction measure for reducing the disease occurrence risk in the human or the animal that is the management target, from the reduction measure memory 306, and specifies the product or the service for implementing the reduction measure.

The health management system according to the embodiment may further include a product/service database 309 for storing a correlation between the reduction measure and the product or the service. For example, the product/service database 309 is included in the computer system. The correlation between the reduction measure and the product or the service is not particularly specified. For example, services such as sports clubs and gyms are registered in the product/service database 309 in relation to exercise as the reduction measure. Furthermore, for example, a smoking cessation aid, a smoking cessation candy, a smoking cessation pipe and the like are registered in the product/service database 309 in relation to smoking cessation as the reduction measure. The product/service database 309 may also store a degree of reliability and/or strength of the correlation between the reduction measure and the product or the service.

For example, the product/service specifier 308 accesses the product/service database 309, and specifies the product or the service for implementing the reduction measure for reducing the disease occurrence risk in the human or the animal that is the management target, based on the reduction measure for reducing the disease occurrence risk in the human or the animal that is the management target and the correlation between the reduction measure and the product or the service. In the case where a plurality of products or services are registered in the product/service database 309 in relation to the reduction measure, the product/service specifier 308 may extract a product or a service with a high degree of reliability and/or strength of correlation to the reduction measure, by excluding a product or a service with a low degree of reliability and/or strength of correlation to the reduction measure.

The health management system according to the embodiment may further include a product/service memory 310 for storing the product or the service, specified by the product/service specifier 308, for implementing the reduction measure for reducing the disease occurrence risk in the human or the animal that is the management target. For example, the product/service memory 310 is included in the computer system. The product/service specifier 308 stores the specified product or service for implementing the reduction measure for reducing the disease occurrence risk in the human or the animal that is the management target, in the product/service memory 310.

The health management system according to the embodiment may further include a product/service information transmitter 311 for transmitting information about the product or the service specified by the product/service specifier 308. For example, the product/service information transmitter 311 is included in the computer system. For example, the product/service information transmitter 311 reads out the product or the service for implementing the reduction measure for reducing the disease occurrence risk in the human or the animal that is the management target, from the product/service memory 310, and transmits information about the product or the service to the manager or the like of the management target human or the management target animal. For example, the product/service information transmitter 311 transmits the information about the product or the service to the display device 405 that is capable of displaying the information about the product or the service to the manager or the like of the management target human or the management target animal.

The risk calculator 301 may receive input of the vital data on the human or the animal subjected to the reduction measure, and may calculate the disease occurrence risk in the human or the animal subjected to the reduction measure. The risk calculator 301 stores the calculated disease occurrence risk in the human or the animal subjected to the reduction measure, in the risk memory 304.

The health management system according to the embodiment may further include a comparator 312 for comparing the disease occurrence risk in the human or the animal before application of the reduction measure, and the disease occurrence risk in the human or the animal after application of the reduction measure. For example, the comparator 312 is included in the computer system. For example, the comparator 312 reads out the disease occurrence risk in the human or the animal before application of the reduction measure and the disease occurrence risk in the human or the animal after application of the reduction measure, from the risk memory 304. Furthermore, for example, the comparator 312 determines whether the disease occurrence risk in the human or the animal after application of the reduction measure is reduced or increased from the disease occurrence risk in the human or the animal before application of the reduction measure.

The health management system according to the embodiment may further include a comparison result memory 313 for storing a comparison result from the comparator 312. For example, the comparison result memory 313 is included in the computer system. The comparator 312 stores the result of comparison between the disease occurrence risk in the human or the animal before application of the reduction measure and the disease occurrence risk in the human or the animal after application of the reduction measure, in the comparison result memory 313.

The health management system according to the embodiment may further include a correlation evaluator 314 for evaluating the correlation between the disease occurrence risk and the reduction measure. For example, the correlation evaluator 314 is included in the computer system. For example, the correlation evaluator 314 reads out the result of comparison between the disease occurrence risk in the human or the animal before application of the reduction measure and the disease occurrence risk in the human or the animal after application of the reduction measure, from the comparison result memory 313. For example, in the case where the disease occurrence risk in the human or the animal after application of the reduction measure is reduced compared to the disease occurrence risk in the human or the animal before application of the reduction measure, the correlation evaluator 314 increases the degree of reliability of the correlation, stored in the reduction measure database 305, between the disease occurrence risk and the reduction measure and/or the strength of the correlation. Furthermore, for example, in the case where the disease occurrence risk in the human or the animal after application of the reduction measure is increased compared to the disease occurrence risk in the human or the animal before application of the reduction measure, the correlation evaluator 314 reduces the degree of reliability of the correlation, stored in the reduction measure database 305, between the disease occurrence risk and the reduction measure and/or the strength of the correlation.

The risk calculator 301 may receive input of the vital data on the human or the animal not subjected to the reduction measure, and calculate the disease occurrence risk in the human or the animal not subjected to the reduction measure. The risk calculator 301 stores the calculated disease occurrence risk in the human or the animal not subjected to the reduction measure, in the risk memory 304.

The comparator 312 may compare the disease occurrence risk in the human or the animal not subjected to the reduction measure with the disease occurrence risk in the human or the animal subjected to the reduction measure. For example, the comparator 312 reads out the disease occurrence risk in the human or the animal not subjected to the reduction measure and the disease occurrence risk in the human or the animal subjected to the reduction measure, from the risk memory 304. Furthermore, for example, the comparator 312 determines whether the disease occurrence risk in the human or the animal subjected to the reduction measure is reduced or increased from the disease occurrence risk in the human or the animal not subjected to the reduction measure.

The comparator 312 stores the result of comparison between the disease occurrence risk in the human or the animal not subjected to the reduction measure and the disease occurrence risk in the human or the animal subjected to the reduction measure, in the comparison result memory 313.

For example, the correlation evaluator 314 reads out the result of comparison between the disease occurrence risk in the human or the animal not subjected to the reduction measure and the disease occurrence risk in the human or the animal subjected to the reduction measure, from the comparison result memory 313. For example, in the case where the disease occurrence risk in the human or the animal subjected to the reduction measure is reduced compared to the disease occurrence risk in the human or the animal not subjected to the reduction measure, the correlation evaluator 314 increases the degree of reliability of the correlation, stored in the reduction measure database 305, between the disease occurrence risk and the reduction measure and/or the strength of the correlation. Furthermore, for example, in the case where the disease occurrence risk in the human or the animal subjected to the reduction measure is increased compared to the disease occurrence risk in the human or the animal not subjected to the reduction measure, the correlation evaluator 314 reduces the degree of reliability of the correlation, stored in the reduction measure database 305, between the disease occurrence risk and the reduction measure and/or the strength of the correlation.

The risk calculator 301 may receive input of the vital data on the human or the animal that used the product or the service, and calculate the disease occurrence risk in the human or the animal that used the product or the service. The risk calculator 301 stores the calculated disease occurrence risk in the human or the animal that used the product or the service, in the risk memory 304.

The comparator 312 may compare the disease occurrence risk in the human or the animal before use of the product or the service with the disease occurrence risk in the human or the animal after use of the product or the service. For example, the comparator 312 reads out the disease occurrence risk in the human or the animal before use of the product or the service and the disease occurrence risk in the human or the animal after use of the product or the service, from the risk memory 304. Furthermore, for example, the comparator 312 determines whether the disease occurrence risk in the human or the animal after use of the product or the service is reduced or increased from the disease occurrence risk in the human or the animal before use of the product or the service. The comparator 312 stores the result of comparison between the disease occurrence risk in the human or the animal before use of the product or the service and the disease occurrence risk in the human or the animal after use of the product or the service, in the comparison result memory 313.

For example, the correlation evaluator 314 reads out the result of comparison between the disease occurrence risk in the human or the animal before use of the product or the service and the disease occurrence risk in the human or the animal after use of the product or the service, from the comparison result memory 313. For example, in the case where the disease occurrence risk in the human or the animal after use of the product or the service is reduced compared to the disease occurrence risk in the human or the animal before use of the product or the service, the correlation evaluator 314 increases the degree of reliability of the correlation, stored in the product/service database 309, between the reduction measure and the product or the service and/or the strength of the correlation. Furthermore, for example, in the case where the disease occurrence risk in the human or the animal after use of the product or the service is increased compared to the disease occurrence risk in the human or the animal before use of the product or the service, the correlation evaluator 314 reduces the degree of reliability of the correlation, stored in the product/service database 309, between the reduction measure and the product or the service and/or the strength of the correlation.

The risk calculator 301 may receive input of the vital data on the human or the animal that did not use the product or the service, and calculate the disease occurrence risk in the human or the animal that did not use the product or the service. The risk calculator 301 stores the calculated disease occurrence risk in the human or the animal that did not use the product or the service, in the risk memory 304.

The comparator 312 may compare the disease occurrence risk in the human or the animal that did not use the product or the service with the disease occurrence risk in the human or the animal that used the product or the service. For example, the comparator 312 reads out the disease occurrence risk in the human or the animal that did not use the product or the service and the disease occurrence risk in the human or the animal that used the product or the service, from the risk memory 304. Furthermore, for example, the comparator 312 determines whether the disease occurrence risk in the human or the animal that used the product or the service is reduced or increased from the disease occurrence risk in the human or the animal that did not use the product or the service. The comparator 312 stores the result of comparison between the disease occurrence risk in the human or the animal that did not use the product or the service and the disease occurrence risk in the human or the animal that used the product or the service, in the comparison result memory 313.

For example, the correlation evaluator 314 reads out the result of comparison between the disease occurrence risk in the human or the animal that did not use the product or the service and the disease occurrence risk in the human or the animal that used the product or the service, from the comparison result memory 313. For example, in the case where the disease occurrence risk in the human or the animal that used the product or the service is reduced compared to the disease occurrence risk in the human or the animal that did not use the product or the service, the correlation evaluator 314 increases the degree of reliability of the correlation, stored in the product/service database 309, between the reduction measure and the product or the service and/or the strength of the correlation. Furthermore, for example, in the case where the disease occurrence risk in the human or the animal that used the product or the service is increased compared to the disease occurrence risk in the human or the animal that did not use the product or the service, the correlation evaluator 314 reduces the degree of reliability of the correlation, stored in the product/service database 309, between the reduction measure and the product or the service and/or the strength of the correlation.

The health management system according to the embodiment may further include a correlation evaluation result transmitter 315 for transmitting an evaluation result regarding the correlation between the reduction measure and the product or the service, to a computer system of a provider of the product or the service. For example, the correlation evaluation result transmitter 315 is included in the computer system. For example, the correlation evaluation result transmitter 315 reads out the correlation between the reduction measure and the product or the service, for which the degree of reliability and/or the strength is increased, from the product/service database 309. Furthermore, the correlation evaluation result transmitter 315 transmits a notification indicating that the degree of reliability of the correlation and/or the strength of the correlation is increased, to the provider of the product or the service. Furthermore, for example, the correlation evaluation result transmitter 315 reads out the correlation between the reduction measure and the product or the service, for which the degree of reliability and/or the strength is reduced, from the product/service database 309. Moreover, the correlation evaluation result transmitter 315 transmits a notification indicating that the degree of reliability of the correlation and/or the strength of the correlation is reduced, to the provider of the product or the service.

With the health management system according to the embodiment described above, a measure for reducing a disease risk, or a product or a service for implementing the measure for reducing the disease risk may be presented in relation to a human or an animal that is a management target, and thus, health may be maintained or the disease occurrence risk may be reduced for the human or the animal that is the management target.

Furthermore, with the health management system according to the embodiment, an evaluation result of the disease occurrence risk in the human or the animal that received the reduction measure, the product or the service may be fed back to the correlation used for specifying the reduction measure, the product or the service. Accordingly, by continuously using the health management system according to the embodiment, accuracy of specifying a measure, a product or a service that is effective in reducing the disease occurrence risk may be increased. Furthermore, with the health management system according to the embodiment, effectiveness of a product or a service may be fed back to the provider of the product or the service.

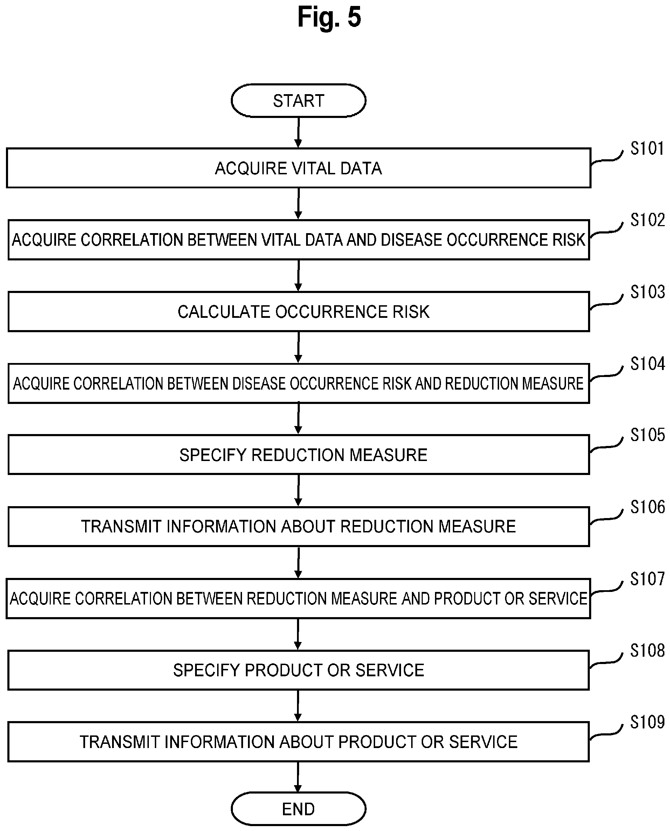

Next, a health management method according to the embodiment will be described with reference to flowcharts shown in Figures 5 to 9. Additionally, the health management method according to the embodiment does not have to include all of the steps described below, and may include additional steps, and order of the steps is not specified.

In step S101 shown in Figure 5, the risk calculator 301 receives input of the vital data on a human or an animal that is a management target. The method used by the risk calculator 301 to receive input of the vital data on the human or the animal is not particularly specified. For example, the risk calculator 301 receives input of the vital data on the human or the animal from the vital data input device 401. Alternatively, the risk calculator 301 receives input of the vital data on the human or the animal from the measure 402. Alternatively, the risk calculator 301 receives input of the vital data on the human or the animal from the vital data memory 403. Alternatively, the risk calculator 301 receives input of the vital data on the human or the animal from the medical facility 502.

In step S102, the risk calculator 302 reads out the correlation between the vital data and the disease occurrence risk, from the disease occurrence risk database 303. In step S103, the risk calculator calculates the disease occurrence risk in the human or the animal that is the management target, based on the input vital data on the human or the animal that is the management target and the read out correlation. The risk calculator 301 stores the calculated disease occurrence risk in the human or the animal that is the management target, in the risk memory 304.

In step S104, the reduction measure specifier 302 reads out the disease occurrence risk in the human or the animal that is the management target, from the risk memory 304. Furthermore, the reduction measure specifier 302 reads out the correlation between the disease occurrence risk and the reduction measure, from the reduction measure database 305. In step S105, the reduction measure specifier 302 specifies the reduction measure for reducing the disease occurrence risk in the human or the animal that is the management target, based on the disease occurrence risk in the human or the animal that is the management target and the correlation between the disease occurrence risk and the reduction measure. The reduction measure specifier 302 stores the specified reduction measure for reducing the disease occurrence risk in the human or the animal that is the management target, in the reduction measure memory 306.

In step S106, the reduction measure information transmitter 307 reads out the reduction measure for reducing the disease occurrence risk in the human or the animal that is the management target, from the reduction measure memory 306. The reduction measure information transmitter 307 transmits information about the reduction measure for reducing the disease occurrence risk, to the manager or the like of the management target human or the management target animal.

In step S107, the product/service specifier 308 reads out the reduction measure for reducing the disease occurrence risk in the human or the animal that is the management target, from the reduction measure memory 306. Furthermore, the product/service specifier 308 reads out the correlation between the reduction measure and the product or the service, from the product/service database 309. In step S108, the product/service specifier 308 specifies the product or the service for implementing the reduction measure for reducing the disease occurrence risk in the human or the animal that is the management target, based on the reduction measure for reducing the disease occurrence risk in the human or the animal that is the management target and the correlation between the reduction measure and the product or the service. The product/service specifier 308 stores the specified product or service in the product/service memory 310.

In step S109, the product/service information transmitter 311 reads out the product or the service for implementing the reduction measure for reducing the disease occurrence risk in the human or the animal that is the management target, from the product/service memory 310. The product/service information transmitter 311 transmits information about the product or the service for implementing the reduction measure for reducing the disease occurrence risk, to the manager or the like of the management target human or the management target animal.

Steps S101 to S106 may be continuously repeated. The vital data may thus be continuously acquired continuously from the human or the animal that is the management target, and information about the reduction measure for reducing the disease occurrence risk may be continuously provided in relation to the human or the animal that is the management target. Furthermore, steps S101 to S109 may be continuously repeated. The vital data may thus be continuously acquired continuously from the human or the animal that is the management target, and information about the product or the service for implementing the reduction measure for reducing the disease occurrence risk may be continuously provided in relation to the human or the animal that is the management target.

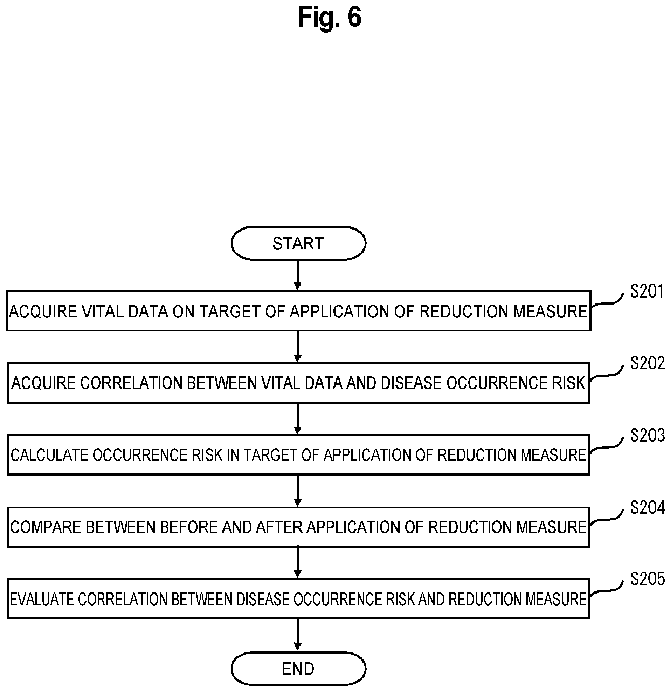

After a lapse of a predetermined period of time from when steps S101 to S106 are first performed, the risk calculator 301 receives, in step S201 shown in Figure 6, input of the vital data on the human or the animal subjected to the reduction measure. The method used by the risk calculator 301 to receive input of the vital data on the human or the animal subjected to the reduction measure is not particularly specified. In step S202, the risk calculator 302 reads out the correlation between the vital data and the disease occurrence risk, from the disease occurrence risk database 303. In step S203, the risk calculator calculates the disease occurrence risk in the human or the animal subjected to the reduction measure, based on the input vital data on the human or the animal subjected to the reduction measure and the read out correlation. The risk calculator 301 stores the calculated disease occurrence risk in the human or the animal subjected to the reduction measure, in the risk memory 304.

In step S204, the comparator 312 reads out the disease occurrence risk in the human or the animal before application of the reduction measure and the disease occurrence risk in the human or the animal after application of the reduction measure, from the risk memory 304. The comparator 312 compares the disease occurrence risk in the human or the animal before application of the reduction measure with the disease occurrence risk in the human or the animal after application of the reduction measure, and determines whether the disease occurrence risk in the human or the animal after application of the reduction measure is reduced or increased from the disease occurrence risk in the human or the animal before application of the reduction measure. The comparator 312 stores the result of comparison between the disease occurrence risk in the human or the animal before application of the reduction measure and the disease occurrence risk in the human or the animal after application of the reduction measure, in the comparison result memory 313.

In step S205, the correlation evaluator 314 reads out the result of comparison between the disease occurrence risk in the human or the animal before application of the reduction measure and the disease occurrence risk in the human or the animal after application of the reduction measure, from the comparison result memory 313. In the case where the disease occurrence risk in the human or the animal after application of the reduction measure is reduced compared to the disease occurrence risk in the human or the animal before application of the reduction measure, the correlation evaluator 314 increases the degree of reliability of the correlation, stored in the reduction measure database 305, between the disease occurrence risk and the reduction measure and/or the strength of the correlation. Furthermore, in the case where the disease occurrence risk in the human or the animal after application of the reduction measure is increased compared to the disease occurrence risk in the human or the animal before application of the reduction measure, the correlation evaluator 314 reduces the degree of reliability of the correlation, stored in the reduction measure database 305, between the disease occurrence risk and the reduction measure and/or the strength of the correlation.

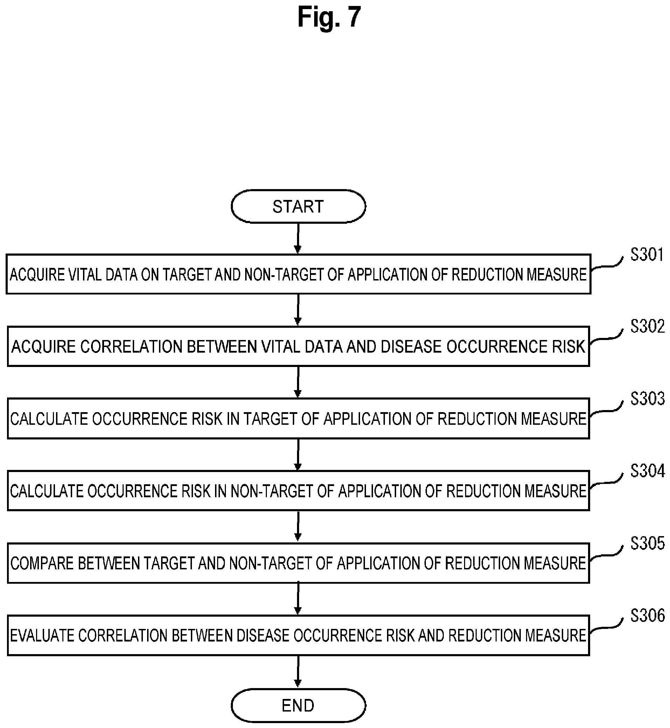

Furthermore, in step S301 shown in Figure 7, the risk calculator 301 receives input of the vital data on the human or the animal subjected to the reduction measure for a predetermined period of time and the vital data on the human or the animal not subjected to the reduction measure for the predetermined period of time. The method used by the risk calculator 301 to receive input of the vital data on the human or the animal subjected to the reduction measure for a predetermined period of time and the vital data on the human or the animal not subjected to the reduction measure for the predetermined period of time is not particularly specified.

In step S302, the risk calculator 302 reads out the correlation between the vital data and the disease occurrence risk, from the disease occurrence risk database 303.

In step S303, the risk calculator calculates the disease occurrence risk of the vital data on the human or the animal subjected to the reduction measure for the predetermined period of time, based on the input vital data on the human or the animal subjected to the reduction measure for the predetermined period of time and the read out correlation. The risk calculator 301 stores the calculated vital data on the human or the animal subjected to the reduction measure for the predetermined period of time, in the risk memory 304.

In step S304, the risk calculator calculates the disease occurrence risk of the vital data on the human or the animal not subjected to the reduction measure for the predetermined period of time, based on the input vital data on the human or the animal not subjected to the reduction measure for the predetermined period of time and the read out correlation. The risk calculator 301 stores the calculated vital data on the human or the animal not subjected to the reduction measure for the predetermined period of time, in the risk memory 304.

In step S305, the comparator 312 reads out the disease occurrence risk in the human or the animal not subjected to the reduction measure for the predetermined period of time and the disease occurrence risk in the human or the animal subjected to the reduction measure for the predetermined period of time, from the risk memory 304. The comparator 312 compares the disease occurrence risk in the human or the animal not subjected to the reduction measure for the predetermined period of time and the disease occurrence risk in the human or the animal subjected to the reduction measure for the predetermined period of time, and determines whether the disease occurrence risk in the human or the animal subjected to the reduction measure for the predetermined period of time is reduced or increased from the disease occurrence risk in the human or the animal not subjected to the reduction measure for the predetermined period of time. The comparator 312 stores the result of comparison between the disease occurrence risk in the human or the animal not subjected to the reduction measure for the predetermined period of time and the disease occurrence risk in the human or the animal subjected to the reduction measure for the predetermined period of time, in the comparison result memory 313.

In step S306, the result of comparison between the disease occurrence risk in the human or the animal not subjected to the reduction measure for the predetermined period of time and the disease occurrence risk in the human or the animal subjected to the reduction measure for the predetermined period of time is read out. In the case where the disease occurrence risk in the human or the animal subjected to the reduction measure for the predetermined period of time is reduced compared to the disease occurrence risk in the human or the animal not subjected to the reduction measure for the predetermined period of time, the correlation evaluator 314 increases the degree of reliability of the correlation, stored in the reduction measure database 305, between the disease occurrence risk and the reduction measure and/or the strength of the correlation. Furthermore, in the case where the disease occurrence risk in the human or the animal subjected to the reduction measure for the predetermined period of time is increased compared to the disease occurrence risk in the human or the animal not subjected to the reduction measure for the predetermined period of time, the correlation evaluator 314 reduces the degree of reliability of the correlation, stored in the reduction measure database 305, between the disease occurrence risk and the reduction measure and/or the strength of the correlation.

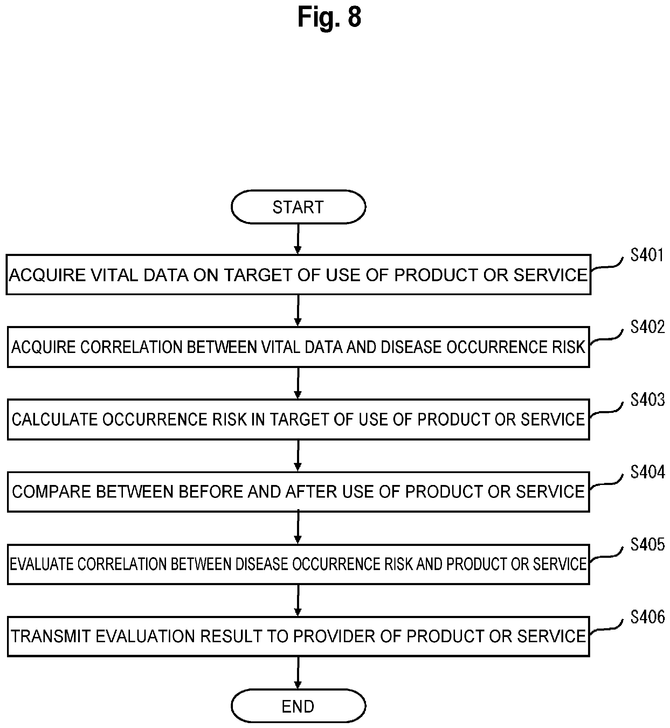

After a lapse of a predetermined period of time from when steps S101 to S109 are first performed, the risk calculator 301 receives, in step S401 shown in Figure 8, input of the vital data on the human or the animal that used the product or the service. The method used by the risk calculator 301 to receive input of the vital data on the human or the animal that used the product or the service is not particularly specified. In step S402, the risk calculator 302 reads out the correlation between the vital data and the disease occurrence risk, from the disease occurrence risk database 303. In step S403, the risk calculator calculates the disease occurrence risk in the human or the animal that used the product or the service, based on the input vital data on the human or the animal that used the product or the service and the read out correlation. The risk calculator 301 stores the calculated disease occurrence risk in the human or the animal that used the product or the service, in the risk memory 304.

In step S404, the comparator 312 reads out the disease occurrence risk in the human or the animal before use of the product or the service and the disease occurrence risk in the human or the animal after use of the product or the service, from the risk memory 304. The comparator 312 compares the disease occurrence risk in the human or the animal before use of the product or the service with the disease occurrence risk in the human or the animal after use of the product or the service, and determines whether the disease occurrence risk in the human or the animal after use of the product or the service is reduced or increased from the disease occurrence risk in the human or the animal before use of the product or the service. The comparator 312 stores the result of comparison between the disease occurrence risk in the human or the animal before use of the product or the service and the disease occurrence risk in the human or the animal after use of the product or the service, in the comparison result memory 313.

In step S405, the correlation evaluator 314 reads out the result of comparison between the disease occurrence risk in the human or the animal before use of the product or the service and the disease occurrence risk in the human or the animal after use of the product or the service, from the comparison result memory 313. In the case where the disease occurrence risk in the human or the animal after use of the product or the service is reduced compared to the disease occurrence risk in the human or the animal before use of the product or the service, the correlation evaluator 314 increases the degree of reliability of the correlation, stored in the product/service database 309, between the reduction measure and the product or the service and/or the strength of the correlation. Furthermore, in the case where the disease occurrence risk in the human or the animal after use of the product or the service is increased compared to the disease occurrence risk in the human or the animal before use of the product or the service, the correlation evaluator 314 reduces the degree of reliability of the correlation, stored in the product/service database 309, between the reduction measure and the product or the service and/or the strength of the correlation.

In step S405, the correlation evaluation result transmitter 315 reads out the correlation between the reduction measure and the product or the service, for which the degree of reliability and/or the strength is increased, from the product/service database 309. Furthermore, the correlation evaluation result transmitter 315 transmits a notification indicating that the degree of reliability of the correlation and/or the strength of the correlation is increased, to the provider of the product or the service. Moreover, the correlation evaluation result transmitter 315 reads out the correlation between the reduction measure and the product or the service, for which the degree of reliability and/or the strength is reduced, from the product/service database 309. Furthermore, the correlation evaluation result transmitter 315 transmits a notification indicating that the degree of reliability of the correlation and/or the strength of the correlation is reduced, to the provider of the product or the service.

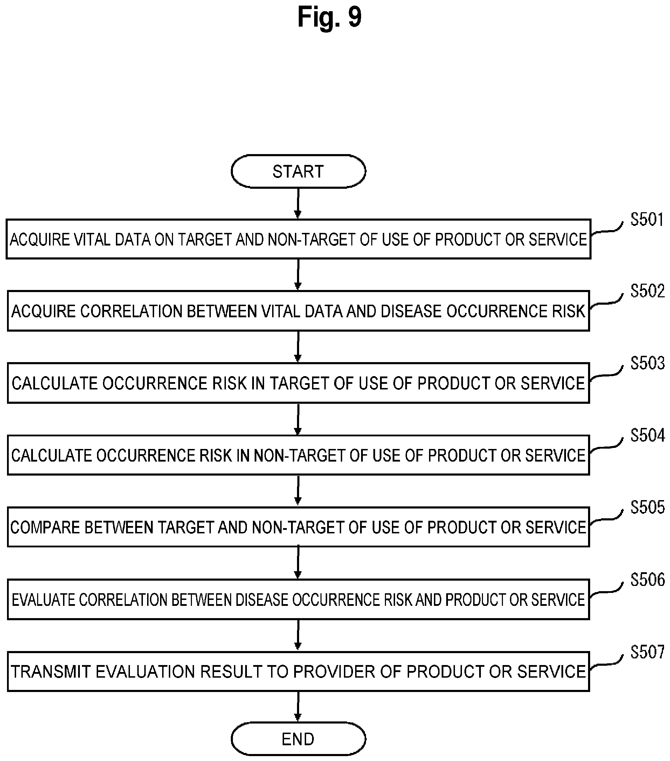

Furthermore, in step S501 shown in Figure 9, the risk calculator 301 receives input of the vital data on the human or the animal that did not use the product or the service for a predetermined period of time and the vital data on the human or the animal that used the product or the service for the predetermined period of time. The method used by the risk calculator 301 to receive input of the vital data on the human or the animal that did not use the product or the service for a predetermined period of time and the vital data on the human or the animal that used the product or the service for the predetermined period of time is not particularly specified.

In step S502, the risk calculator 302 reads out the correlation between the vital data and the disease occurrence risk, from the disease occurrence risk database 303.

In step S503, the risk calculator calculates the disease occurrence risk of the vital data on the human or the animal that used the product or the service for the predetermined period of time, based on the input vital data on the human or the animal that used the product or the service for the predetermined period of time and the read out correlation. The risk calculator 301 stores the calculated vital data on the human or the animal that used the product or the service for the predetermined period of time, in the risk memory 304.

In step S504, the risk calculator calculates the disease occurrence risk of the vital data on the human or the animal that did not use the product or the service for the predetermined period of time, based on the input vital data on the human or the animal that did not use the product or the service for the predetermined period of time and the read out correlation. The risk calculator 301 stores the calculated vital data on the human or the animal that did not use the product or the service for the predetermined period of time, in the risk memory 304.

In step S505, the comparator 312 reads out the disease occurrence risk in the human or the animal that did not use the product or the service for the predetermined period of time and the disease occurrence risk in the human or the animal that used the product or the service for the predetermined period of time, from the risk memory 304. The comparator 312 compares the disease occurrence risk in the human or the animal that did not use the product or the service for the predetermined period of time and the disease occurrence risk in the human or the animal that used the product or the service for the predetermined period of time, and determines whether the disease occurrence risk in the human or the animal that used the product or the service for the predetermined period of time is reduced or increased from the disease occurrence risk in the human or the animal that did not use the product or the service for the predetermined period of time. The comparator 312 stores the result of comparison between the disease occurrence risk in the human or the animal that did not use the product or the service for the predetermined period of time and the disease occurrence risk in the human or the animal that used the product or the service for the predetermined period of time, in the comparison result memory 313.

In step S506, the result of comparison between the disease occurrence risk in the human or the animal that did not use the product or the service for the predetermined period of time and the disease occurrence risk in the human or the animal that used the product or the service for the predetermined period of time is read out. In the case where the disease occurrence risk in the human or the animal that used the product or the service for the predetermined period of time is reduced compared to the disease occurrence risk in the human or the animal that did not use the product or the service for the predetermined period of time, the correlation evaluator 314 increases the degree of reliability of the correlation, stored in the product/service database 309, between the reduction measure and the product or the service and/or the strength of the correlation. Furthermore, in the case where the disease occurrence risk in the human or the animal that used the product or the service for the predetermined period of time is increased compared to the disease occurrence risk in the human or the animal that did not use the product or the service for the predetermined period of time, the correlation evaluator 314 reduces the degree of reliability of the correlation, stored in the product/service database 309, between the reduction measure and the product or the service and/or the strength of the correlation.

In step S507, the correlation evaluation result transmitter 315 reads out the correlation between the reduction measure and the product or the service, for which the degree of reliability and/or the strength is increased, from the product/service database 309. Furthermore, the correlation evaluation result transmitter 315 transmits a notification indicating that the degree of reliability of the correlation and/or the strength of the correlation is increased, to the provider of the product or the service. Moreover, the correlation evaluation result transmitter 315 reads out the correlation between the reduction measure and the product or the service, for which the degree of reliability and/or the strength is reduced, from the product/service database 309. Furthermore, the correlation evaluation result transmitter 315 transmits a notification indicating that the degree of reliability of the correlation and/or the strength of the correlation is reduced, to the provider of the product or the service.

The present invention has been described according to the embodiment as described above, but the descriptions and drawings forming a part of the disclosure should not be understood to limit the present invention. Various alternate embodiments, examples and operational technologies should be obvious to those skilled in the art based on the disclosure. The present invention should be understood to include various embodiments not described herein.

Reference Signs List

| | |
|---|---|
| 300 | computer system |
| 301 | risk calculator |
| 302 | reduction measure specifier |
| 303 | disease occurrence risk database |
| 304 | risk memory |
| 305 | reduction measure database |
| 306 | reduction measure memory |
| 307 | reduction measure information transmitter |
| 308 | product/service specifier |
| 309 | product/service database |
| 310 | product/service memory |
| 311 | product/service information transmitter |
| 312 | comparator |
| 313 | comparison result memory |
| 314 | correlation evaluator |
| 315 | correlation evaluation result transmitter |
| 401 | vital data input device |
| 402 | measure |
| 403 | vital data memory |
| 404 | computer system |
| 405 | display device |
| 501 | building |
| 502 | medical facility |

**Claims**

1. A health management system comprising:

   a risk calculator for receiving input of vital data on a human or an animal, and for calculating a disease occurrence risk in the human or the animal; and
   a reduction measure specifier for specifying a reduction measure for reducing the disease occurrence risk in the human or the animal.

2. The health management system according to claim 1, further comprising a measure for measuring the vital data.

3. The health management system according to claim 2, wherein the measure is disposed in a building where the human or the animal is present.

4. The health management system according to claim 2, wherein the measure is attached to the human or the animal.

5. The health management system according to any one of claims 1 to 4, further comprising an input device for receiving input of the vital data from the human, and for transmitting the vital data to the risk calculator.

6. The health management system according to any one of claims 1 to 5, wherein the risk calculator receives input of the vital data on the human or the animal from a medical facility.

7. The health management system according to any one of claims 1 to 6, further comprising a reduction measure information transmitter for transmitting information about the reduction measure that is specified.

8. The health management system according to any one of claims 1 to 7, further comprising a disease occurrence risk database for storing a correlation between the vital data and the disease occurrence risk, wherein
the risk calculator calculates the disease occurrence risk by accessing the disease occurrence risk database.

9. The health management system according to any one of claims 1 to 8, further comprising a reduction measure database for storing a correlation between the disease occurrence risk and the reduction measure, wherein
the reduction measure specifier specifies the reduction measure by accessing the reduction measure database.

10. The health management system according to any one of claims 1 to 8, wherein the risk calculator receives input of the vital data on the human or the animal subjected to the reduction measure, and calculates the disease occurrence risk in the human or the animal subjected to the reduction measure.

11. The health management system according to claim 10, further comprising a comparator for comparing the disease occurrence risk in the human or the animal before application of the reduction measure, and the disease occurrence risk in the human or the animal after application of the reduction measure.

12. The health management system according to claim 11, further comprising a reduction measure database for storing a correlation between the disease occurrence risk and the reduction measure, wherein

the reduction measure specifier specifies the reduction measure by accessing the reduction measure database, and
the health management system further comprises a correlation evaluator for increasing a degree of reliability of the correlation between the disease occurrence risk and the reduction measure and/or strength of the correlation in a case where the disease occurrence risk in the human or the animal after application of the reduction measure is reduced compared to the disease occurrence risk in the human or the animal before application of the reduction measure.

13. The health management system according to claim 12, wherein, in a case where the disease occurrence risk in the human or the animal after application of the reduction measure is increased compared to the disease occurrence risk in the human or the animal before application of the reduction measure, the correlation evaluator reduces the degree of reliability of the correlation between the disease occurrence risk and the reduction measure and/or the strength of the correlation.

14. The health management system according to any one of claims 1 to 8, wherein the risk calculator receives input of the vital data on the human or the animal not subjected to the reduction measure and the vital data on the human or the animal subjected to the reduction measure, and calculates the disease occurrence risk in the human or the animal not subjected to the reduction measure and the disease occurrence risk in the human or the animal subjected to the reduction measure.

15. The health management system according to claim 14, further comprising a comparator for comparing the disease occurrence risk in the human or the animal not subjected to the reduction measure, and the disease occurrence risk in the human or the animal subjected to the reduction measure.

16. The health management system according to claim 15, further comprising a reduction measure database for storing a correlation between the disease occurrence risk and the reduction measure, wherein

the reduction measure specifier specifies the reduction measure by accessing the reduction measure database, and
the health management system further comprises a correlation evaluator for increasing a degree of reliability of the correlation between the disease occurrence risk and the reduction measure and/or strength of the correlation in a case where the disease occurrence risk in the human or the animal subjected to the reduction measure is reduced compared to the disease occurrence risk in the human or the animal not subjected to the reduction measure.

17. The health management system according to claim 16, wherein, in a case where the disease occurrence risk in the human or the animal subjected to the reduction measure is increased compared to the disease occurrence risk in the human or the animal not subjected to the reduction measure, the correlation evaluator reduces the degree of reliability of the correlation between the disease occurrence risk and the reduction measure and/or the strength of the correlation.

18. The health management system according to any one of claims 1 to 9, further comprising a product/service specifier for specifying a product or a service for implementing the reduction measure.

19. The health management system according to claim 18, further comprising a product/service information transmitter for transmitting information about the product or the service that is specified.

20. The health management system according to claim 18 or 19, further comprising a product/service database for storing a correlation between the reduction measure and the product or the service, wherein
the product/service specifier specifies the product or the service by accessing the product/service database.

21. The health management system according to claim 18 or 19, wherein the risk calculator receives input of the vital data on the human or the animal that used the product or the service, and calculates the disease occurrence risk in the human or the animal that used the product or the service.

22. The health management system according to claim 21, further comprising a comparator for comparing the disease occurrence risk in the human or the animal before use of the product or the service, and the disease occurrence risk in the human or the animal after use of the product or the service.

23. The health management system according to claim 22, further comprising a product/service database for storing a correlation between the reduction measure and the product or the service, wherein

the product/service specifier specifies the product or the service by accessing the product/service database, and the health management system further comprises a correlation evaluator for increasing a degree of reliability of the correlation between the reduction measure and the product or the service and/or strength of the correlation in a case where the disease occurrence risk in the human or the animal after use of the product or the service is reduced compared to the disease occurrence risk in the human or the animal before use of the product or the service.

24. The health management system according to claim 23, further comprising a correlation evaluation result transmitter for transmitting a notification indicating that the degree of reliability of the correlation and/or the strength of the correlation is increased, to a provider of the product or the service.

25. The health management system according to claim 23, wherein, in a case where the disease occurrence risk in the human or the animal after use of the product or the service is increased compared to the disease occurrence risk in the human or the animal before use of the product or the service, the correlation evaluator reduces the degree of reliability of the correlation between the reduction measure and the product or the service and/or the strength of the correlation.

26. The health management system according to claim 25, further comprising a correlation evaluation result transmitter for transmitting a notification indicating that the degree of reliability of the correlation and/or the strength of the correlation is reduced, to a provider of the product or the service.

27. The health management system according to claim 18 or 19, wherein the risk calculator receives input of the vital data on the human or the animal that did not use the product or the service and the vital data on the human or the animal that used the product or the service, and calculates the disease occurrence risk in the human or the animal that did not use the product or the service and the disease occurrence risk in the human or the animal that used the product or the service.

**28.** The health management system according to claim 27, further comprising a comparator for comparing the disease occurrence risk in the human or the animal that did not use the product or the service, and the disease occurrence risk in the human or the animal that used the product or the service.

**29.** The health management system according to claim 28, further comprising a product/service database for storing a correlation between the reduction measure and the product or the service, wherein

the product/service specifier specifies the product or the service by accessing the product/service database, and the health management system further comprises a correlation evaluator for increasing a degree of reliability of the correlation between the reduction measure and the product or the service and/or strength of the correlation in a case where the disease occurrence risk in the human or the animal that used the product or the service is reduced compared to the disease occurrence risk in the human or the animal that did not use the product or the service.

**30.** The health management system according to claim 29, further comprising a correlation evaluation result transmitter for transmitting a notification indicating that the degree of reliability of the correlation and/or the strength of the correlation is increased, to a provider of the product or the service.

**31.** The health management system according to claim 29 or 30, wherein, in a case where the disease occurrence risk in the human or the animal that used the product or the service is increased compared to the disease occurrence risk in the human or the animal that did not use the product or the service, the correlation evaluator reduces the degree of reliability of the correlation between the reduction measure and the product or the service and/or the strength of the correlation.

**32.** The health management system according to claim 31, further comprising a correlation evaluation result transmitter for transmitting a notification indicating that the degree of reliability of the correlation and/or the strength of the correlation is reduced, to a provider of the product or the service.

**33.** The health management system according to any one of claims 1 to 32, wherein the vital data includes at least one of sex, age, height, and weight of the human or the animal.

**34.** The health management system according to any one of claims 1 to 33, wherein the vital data includes at least one of a past medical history and a family medical history of the human or the animal.

**35.** The health management system according to any one of claims 1 to 34, wherein the vital data includes at least one of a heart rate, respiration, a body movement, a body temperature, an ambient temperature, and an ambient humidity of the human or the animal.

**36.** The health management system according to any one of claims 1 to 35, wherein the vital data includes at least one of blood pressure, a biochemical test result, and a hematology test result of the human or the animal.

**37.** The health management system according to any one of claims 1 to 36, wherein the vital data includes an ingested substance of the human or the animal.

**38.** The health management system according to any one of claims 1 to 37, wherein the vital data includes a medical treatment received by the human or the animal, and a medication administration history.

**39.** The health management system according to any one of claims 1 to 38, wherein the vital data includes genetic information on the human or the animal.

**40.** A health management method comprising:

receiving input of vital data on a human or an animal, and calculating a disease occurrence risk in the human or the animal, the receiving and the calculating being performed by a risk calculator of a computer system; and specifying, by a reduction measure specifier of the computer system, a reduction measure for reducing the disease occurrence risk in the human or the animal.

**41.** The health management method according to claim 40, further comprising measuring the vital data by a measure.

42. The health management method according to claim 41, wherein the measure is disposed in a building where the human or the animal is present.

43. The health management method according to claim 41, wherein the measure is attached to the human or the animal.

44. The health management method according to any one of claims 40 to 43, further comprising receiving input of the vital data from the human, and transmitting the vital data to the risk calculator, the receiving and the transmitting being performed by an input device.

45. The health management method according to any one of claims 40 to 44, further comprising receiving, by the risk calculator, input of the vital data on the human or the animal from a medical facility.

46. The health management method according to any one of claims 40 to 45, further comprising transmitting, by a reduction measure information transmitter of the computer system, information about the reduction measure that is specified.

47. The health management method according to any one of claims 40 to 46, further comprising preparing a disease occurrence risk database for storing a correlation between the vital data and the disease occurrence risk, wherein the risk calculator calculates the disease occurrence risk by accessing the disease occurrence risk database.

48. The health management method according to any one of claims 40 to 47, further comprising preparing a reduction measure database for storing a correlation between the disease occurrence risk and the reduction measure, wherein the reduction measure specifier specifies the reduction measure by accessing the reduction measure database.

49. The health management method according to any one of claims 40 to 47, further comprising receiving input of the vital data on the human or the animal subjected to the reduction measure, and calculating the disease occurrence risk in the human or the animal subjected to the reduction measure, the receiving and the calculating being performed by the risk calculator.

50. The health management method according to claim 49, further comprising comparing, by a comparator of the computer system, the disease occurrence risk in the human or the animal before application of the reduction measure, and the disease occurrence risk in the human or the animal after application of the reduction measure.

51. The health management method according to claim 50, further comprising:

preparing a reduction measure database for storing a correlation between the disease occurrence risk and the reduction measure;
specifying, by the reduction measure specifier, the reduction measure by accessing the reduction measure database; and
increasing, by a correlation evaluator of the computer system, a degree of reliability of the correlation between the disease occurrence risk and the reduction measure and/or strength of the correlation in a case where the disease occurrence risk in the human or the animal after application of the reduction measure is reduced compared to the disease occurrence risk in the human or the animal before application of the reduction measure.

52. The health management method according to claim 51, further comprising reducing, by the correlation evaluator, the degree of reliability of the correlation between the disease occurrence risk and the reduction measure and/or the strength of the correlation in a case where the disease occurrence risk in the human or the animal after application of the reduction measure is increased compared to the disease occurrence risk in the human or the animal before application of the reduction measure.

53. The health management method according to any one of claims 40 to 47, further comprising receiving input of the vital data on the human or the animal not subjected to the reduction measure and the vital data on the human or the animal subjected to the reduction measure, and calculating the disease occurrence risk in the human or the animal not subjected to the reduction measure and the disease occurrence risk in the human or the animal subjected to the reduction measure, the receiving and the calculating being performed by the risk calculator.

54. The health management method according to claim 53, further comprising comparing, by a comparator of the computer system, the disease occurrence risk in the human or the animal not subjected to the reduction measure,

and the disease occurrence risk in the human or the animal subjected to the reduction measure.

55. The health management method according to claim 54, further comprising:

preparing a reduction measure database for storing a correlation between the disease occurrence risk and the reduction measure;
specifying, by the reduction measure specifier, the reduction measure by accessing the reduction measure database; and
increasing, by a correlation evaluator of the computer system, a degree of reliability of the correlation between the disease occurrence risk and the reduction measure and/or strength of the correlation in a case where the disease occurrence risk in the human or the animal subjected to the reduction measure is reduced compared to the disease occurrence risk in the human or the animal not subjected to the reduction measure.

56. The health management method according to claim 55, further comprising reducing, by the correlation evaluator, the degree of reliability of the correlation between the disease occurrence risk and the reduction measure and/or the strength of the correlation in a case where the disease occurrence risk in the human or the animal subjected to the reduction measure is increased compared to the disease occurrence risk in the human or the animal not subjected to the reduction measure.

57. The health management method according to any one of claims 40 to 48, further comprising specifying, by a product/service specifier of the computer system, a product or a service for implementing the reduction measure.

58. The health management method according to claim 57, further comprising transmitting, by a product/service information transmitter of the computer system, information about the product or the service that is specified.

59. The health management method according to claim 57 or 58, further comprising preparing a product/service database for storing a correlation between the reduction measure and the product or the service, wherein
the product/service specifier specifies the product or the service by accessing the product/service database.

60. The health management method according to claim 57 or 58, further comprising receiving input of the vital data on the human or the animal that used the product or the service, and calculating the disease occurrence risk in the human or the animal that used the product or the service, the receiving and the calculating being performed by the risk calculator.

61. The health management method according to claim 60, further comprising comparing, by a comparator of the computer system, the disease occurrence risk in the human or the animal before use of the product or the service, and the disease occurrence risk in the human or the animal after use of the product or the service.

62. The health management method according to claim 61, further comprising:

preparing a product/service database for storing a correlation between the reduction measure and the product or the service;
specifying, by the product/service specifier, the product or the service by accessing the product/service database; and
increasing, by a correlation evaluator of the computer system, a degree of reliability of the correlation between the reduction measure and the product or the service and/or strength of the correlation in a case where the disease occurrence risk in the human or the animal after use of the product or the service is reduced compared to the disease occurrence risk in the human or the animal before use of the product or the service.

63. The health management method according to claim 62, further comprising transmitting, by a correlation evaluation result transmitter of the computer system, a notification indicating that the degree of reliability of the correlation and/or the strength of the correlation is increased, to a provider of the product or the service.

64. The health management method according to claim 62 or 63, further comprising reducing, by the correlation evaluator, the degree of reliability of the correlation between the reduction measure and the product or the service and/or the strength of the correlation in a case where the disease occurrence risk in the human or the animal after use of the product or the service is increased compared to the disease occurrence risk in the human or the animal before use of the product or the service.

**65.** The health management method according to claim 64, further comprising transmitting, by a correlation evaluation result transmitter of the computer system, a notification indicating that the degree of reliability of the correlation and/or the strength of the correlation is reduced, to a provider of the product or the service.

**66.** The health management method according to claim 57 or 58, further comprising receiving input of the vital data on the human or the animal that did not use the product or the service and the vital data on the human or the animal that used the product or the service, and calculating the disease occurrence risk in the human or the animal that did not use the product or the service and the disease occurrence risk in the human or the animal that used the product or the service, the receiving and the calculating being performed by the risk calculator.

**67.** The health management method according to claim 66, further comprising comparing, by a comparator of the computer system, the disease occurrence risk in the human or the animal that did not use the product or the service, and the disease occurrence risk in the human or the animal that used the product or the service.

**68.** The health management method according to claim 67, further comprising:

preparing a product/service database for storing a correlation between the reduction measure and the product or the service;
specifying, by the product/service specifier, the product or the service by accessing the product/service database; and
increasing, by a correlation evaluator of the computer system, a degree of reliability of the correlation between the reduction measure and the product or the service and/or strength of the correlation in a case where the disease occurrence risk in the human or the animal that used the product or the service is reduced compared to the disease occurrence risk in the human or the animal that did not use the product or the service.

**69.** The health management method according to claim 68, further comprising transmitting, by a correlation evaluation result transmitter of the computer system, a notification indicating that the degree of reliability of the correlation and/or the strength of the correlation is increased, to a provider of the product or the service.

**70.** The health management method according to claim 68 or 69, further comprising reducing, by the correlation evaluator, the degree of reliability of the correlation between the reduction measure and the product or the service and/or the strength of the correlation in a case where the disease occurrence risk in the human or the animal that used the product or the service is increased compared to the disease occurrence risk in the human or the animal that did not use the product or the service.

**71.** The health management method according to claim 70, further comprising transmitting, by a correlation evaluation result transmitter of the computer system, a notification indicating that the degree of reliability of the correlation and/or the strength of the correlation is reduced, to a provider of the product or the service.

**72.** The health management method according to any one of claims 40 to 71, wherein the vital data includes at least one of sex, age, height, and weight of the human or the animal.

**73.** The health management method according to any one of claims 40 to 72, wherein the vital data includes at least one of a past medical history and a family medical history of the human or the animal.

**74.** The health management method according to any one of claims 40 to 73, wherein the vital data includes at least one of a heart rate, respiration, a body movement, a body temperature, an ambient temperature, and an ambient humidity of the human or the animal.

**75.** The health management method according to any one of claims 40 to 74, wherein the vital data includes at least one of blood pressure, a biochemical test result, and a hematology test result of the human or the animal.

**76.** The health management method according to any one of claims 40 to 75, wherein the vital data includes an ingested substance of the human or the animal.

**77.** The health management method according to any one of claims 40 to 76, wherein the vital data includes a medical treatment received by the human or the animal, and a medication administration history.

**78.** The health management method according to any one of claims 40 to 77, wherein the vital data includes genetic information on the human or the animal.

EP 4 084 012 A1

## Fig. 1

COMPUTER SYSTEM — 300

VITAL DATA INPUT DEVICE — 401

DISPLAY DEVICE — 405

RISK CALCULATOR — 301

REDUCTION MEASURE SPECIFIER — 302

DISEASE OCCURRENCE RISK DATABASE — 303

RISK MEMORY — 304

REDUCTION MEASURE DATABASE — 305

REDUCTION MEASURE MEMORY — 306

REDUCTION MEASURE INFORMATION TRANSMITTER — 307

PRODUCT/SERVICE SPECIFIER — 308

PRODUCT/SERVICE DATABASE — 309

PRODUCT/SERVICE MEMORY — 310

PRODUCT/SERVICE INFORMATION TRANSMITTER — 311

COMPARATOR — 312

COMPARISON RESULT MEMORY — 313

CORRELATION EVALUATOR — 314

CORRELATION EVALUATION RESULT TRANSMITTER — 315

## Fig. 2

COMPUTER SYSTEM — 300

| 301 RISK CALCULATOR |
| 302 REDUCTION MEASURE SPECIFIER |
| 303 DISEASE OCCURRENCE RISK DATABASE |
| 304 RISK MEMORY |
| 305 REDUCTION MEASURE DATABASE |
| 306 REDUCTION MEASURE MEMORY |
| 307 REDUCTION MEASURE INFORMATION TRANSMITTER |
| 308 PRODUCT/SERVICE SPECIFIER |
| 309 PRODUCT/SERVICE DATABASE |
| 310 PRODUCT/SERVICE MEMORY |

| 311 PRODUCT/SERVICE INFORMATION TRANSMITTER |
| 312 COMPARATOR |
| 313 COMPARISON RESULT MEMORY |
| 314 CORRELATION EVALUATOR |
| 315 CORRELATION EVALUATION RESULT TRANSMITTER |

501

402 MEASURE

405 DISPLAY DEVICE

EP 4 084 012 A1

## Fig. 3

VITAL DATA MEMORY — 403

COMPUTER SYSTEM — 300

RISK CALCULATOR — 301

REDUCTION MEASURE SPECIFIER — 302

DISEASE OCCURRENCE RISK DATABASE — 303

RISK MEMORY — 304

REDUCTION MEASURE DATABASE — 305

REDUCTION MEASURE MEMORY — 306

REDUCTION MEASURE INFORMATION TRANSMITTER — 307

PRODUCT/SERVICE SPECIFIER — 308

PRODUCT/SERVICE DATABASE — 309

PRODUCT/SERVICE MEMORY — 310

PRODUCT/SERVICE INFORMATION TRANSMITTER — 311

COMPARATOR — 312

COMPARISON RESULT MEMORY — 313

CORRELATION EVALUATOR — 314

CORRELATION EVALUATION RESULT TRANSMITTER — 315

EP 4 084 012 A1

## Fig. 4

COMPUTER SYSTEM — 300

RISK CALCULATOR — 301

PRODUCT/SERVICE INFORMATION TRANSMITTER — 311

REDUCTION MEASURE SPECIFIER — 302

COMPARATOR — 312

DISEASE OCCURRENCE RISK DATABASE — 303

COMPARISON RESULT MEMORY — 313

RISK MEMORY — 304

CORRELATION EVALUATOR — 314

REDUCTION MEASURE DATABASE — 305

CORRELATION EVALUATION RESULT TRANSMITTER — 315

REDUCTION MEASURE MEMORY — 306

REDUCTION MEASURE INFORMATION TRANSMITTER — 307

PRODUCT/SERVICE SPECIFIER — 308

PRODUCT/SERVICE DATABASE — 309

PRODUCT/SERVICE MEMORY — 310

COMPUTER SYSTEM — 502

404

EP 4 084 012 A1

# Fig. 5

```
        ┌─────────────┐
        │    START    │
        └─────────────┘
               │
               ▼
┌───────────────────────────────────────────────────────────┐
│              ACQUIRE VITAL DATA                             │  S101
└───────────────────────────────────────────────────────────┘
               │
               ▼
┌───────────────────────────────────────────────────────────┐
│ ACQUIRE CORRELATION BETWEEN VITAL DATA AND DISEASE OCCURRENCE RISK │  S102
└───────────────────────────────────────────────────────────┘
               │
               ▼
┌───────────────────────────────────────────────────────────┐
│             CALCULATE OCCURRENCE RISK                       │  S103
└───────────────────────────────────────────────────────────┘
               │
               ▼
┌───────────────────────────────────────────────────────────┐
│ ACQUIRE CORRELATION BETWEEN DISEASE OCCURRENCE RISK AND REDUCTION MEASURE │  S104
└───────────────────────────────────────────────────────────┘
               │
               ▼
┌───────────────────────────────────────────────────────────┐
│             SPECIFY REDUCTION MEASURE                       │  S105
└───────────────────────────────────────────────────────────┘
               │
               ▼
┌───────────────────────────────────────────────────────────┐
│       TRANSMIT INFORMATION ABOUT REDUCTION MEASURE          │  S106
└───────────────────────────────────────────────────────────┘
               │
               ▼
┌───────────────────────────────────────────────────────────┐
│ ACQUIRE CORRELATION BETWEEN REDUCTION MEASURE AND PRODUCT OR SERVICE │  S107
└───────────────────────────────────────────────────────────┘
               │
               ▼
┌───────────────────────────────────────────────────────────┐
│             SPECIFY PRODUCT OR SERVICE                      │  S108
└───────────────────────────────────────────────────────────┘
               │
               ▼
┌───────────────────────────────────────────────────────────┐
│       TRANSMIT INFORMATION ABOUT PRODUCT OR SERVICE         │  S109
└───────────────────────────────────────────────────────────┘
               │
               ▼
        ┌─────────────┐
        │     END     │
        └─────────────┘
```

# Fig. 6

```
                    ┌──────────────┐
                    │    START     │
                    └──────┬───────┘
                           ▼
┌─────────────────────────────────────────────────────────────┐       S201
│ ACQUIRE VITAL DATA ON TARGET OF APPLICATION OF REDUCTION     │
│ MEASURE                                                      │
└─────────────────────────────────────────────────────────────┘
                           ▼
┌─────────────────────────────────────────────────────────────┐       S202
│ ACQUIRE CORRELATION BETWEEN VITAL DATA AND DISEASE           │
│ OCCURRENCE RISK                                              │
└─────────────────────────────────────────────────────────────┘
                           ▼
┌─────────────────────────────────────────────────────────────┐       S203
│ CALCULATE OCCURRENCE RISK IN TARGET OF APPLICATION OF        │
│ REDUCTION MEASURE                                            │
└─────────────────────────────────────────────────────────────┘
                           ▼
┌─────────────────────────────────────────────────────────────┐       S204
│ COMPARE BETWEEN BEFORE AND AFTER APPLICATION OF REDUCTION    │
│ MEASURE                                                      │
└─────────────────────────────────────────────────────────────┘
                           ▼
┌─────────────────────────────────────────────────────────────┐       S205
│ EVALUATE CORRELATION BETWEEN DISEASE OCCURRENCE RISK AND     │
│ REDUCTION MEASURE                                            │
└─────────────────────────────────────────────────────────────┘
                           ▼
                    ┌──────────────┐
                    │     END      │
                    └──────────────┘
```

# Fig. 7

```
                        ┌─────────────────┐
                        │      START      │
                        └─────────────────┘
                                 │
                                 ▼
┌──────────────────────────────────────────────────────────────────────┐        S301
│ ACQUIRE VITAL DATA ON TARGET AND NON-TARGET OF APPLICATION OF REDUCTION MEASURE │
└──────────────────────────────────────────────────────────────────────┘
                                 │
                                 ▼
┌──────────────────────────────────────────────────────────────────────┐        S302
│ ACQUIRE CORRELATION BETWEEN VITAL DATA AND DISEASE OCCURRENCE RISK      │
└──────────────────────────────────────────────────────────────────────┘
                                 │
                                 ▼
┌──────────────────────────────────────────────────────────────────────┐        S303
│ CALCULATE OCCURRENCE RISK IN TARGET OF APPLICATION OF REDUCTION MEASURE │
└──────────────────────────────────────────────────────────────────────┘
                                 │
                                 ▼
┌──────────────────────────────────────────────────────────────────────┐        S304
│ CALCULATE OCCURRENCE RISK IN NON-TARGET OF APPLICATION OF REDUCTION MEASURE │
└──────────────────────────────────────────────────────────────────────┘
                                 │
                                 ▼
┌──────────────────────────────────────────────────────────────────────┐        S305
│ COMPARE BETWEEN TARGET AND NON-TARGET OF APPLICATION OF REDUCTION MEASURE │
└──────────────────────────────────────────────────────────────────────┘
                                 │
                                 ▼
┌──────────────────────────────────────────────────────────────────────┐        S306
│ EVALUATE CORRELATION BETWEEN DISEASE OCCURRENCE RISK AND REDUCTION MEASURE │
└──────────────────────────────────────────────────────────────────────┘
                                 │
                                 ▼
                        ┌─────────────────┐
                        │       END       │
                        └─────────────────┘
```

# Fig. 8

START

ACQUIRE VITAL DATA ON TARGET OF USE OF PRODUCT OR SERVICE — S401

ACQUIRE CORRELATION BETWEEN VITAL DATA AND DISEASE OCCURRENCE RISK — S402

CALCULATE OCCURRENCE RISK IN TARGET OF USE OF PRODUCT OR SERVICE — S403

COMPARE BETWEEN BEFORE AND AFTER USE OF PRODUCT OR SERVICE — S404

EVALUATE CORRELATION BETWEEN DISEASE OCCURRENCE RISK AND PRODUCT OR SERVICE — S405

TRANSMIT EVALUATION RESULT TO PROVIDER OF PRODUCT OR SERVICE — S406

END

# Fig. 9

```
                        ┌──────────────┐
                        │    START     │
                        └──────┬───────┘
                               │
  ┌────────────────────────────▼──────────────────────────────┐  ╭ S501
  │ ACQUIRE VITAL DATA ON TARGET AND NON-TARGET OF USE OF PRODUCT OR SERVICE │
  └────────────────────────────┬──────────────────────────────┘
                               │
  ┌────────────────────────────▼──────────────────────────────┐  ╭ S502
  │ ACQUIRE CORRELATION BETWEEN VITAL DATA AND DISEASE OCCURRENCE RISK │
  └────────────────────────────┬──────────────────────────────┘
                               │
  ┌────────────────────────────▼──────────────────────────────┐  ╭ S503
  │ CALCULATE OCCURRENCE RISK IN TARGET OF USE OF PRODUCT OR SERVICE │
  └────────────────────────────┬──────────────────────────────┘
                               │
  ┌────────────────────────────▼──────────────────────────────┐  ╭ S504
  │ CALCULATE OCCURRENCE RISK IN NON-TARGET OF USE OF PRODUCT OR SERVICE │
  └────────────────────────────┬──────────────────────────────┘
                               │
  ┌────────────────────────────▼──────────────────────────────┐  ╭ S505
  │ COMPARE BETWEEN TARGET AND NON-TARGET OF USE OF PRODUCT OR SERVICE │
  └────────────────────────────┬──────────────────────────────┘
                               │
  ┌────────────────────────────▼──────────────────────────────┐  ╭ S506
  │ EVALUATE CORRELATION BETWEEN DISEASE OCCURRENCE RISK AND PRODUCT OR SERVICE │
  └────────────────────────────┬──────────────────────────────┘
                               │
  ┌────────────────────────────▼──────────────────────────────┐  ╭ S507
  │ TRANSMIT EVALUATION RESULT TO PROVIDER OF PRODUCT OR SERVICE │
  └────────────────────────────┬──────────────────────────────┘
                               │
                        ┌──────▼───────┐
                        │     END      │
                        └──────────────┘
```

## INTERNATIONAL SEARCH REPORT

| | International application No. |
|---|---|
| | PCT/JP2020/048146 |

**A. CLASSIFICATION OF SUBJECT MATTER**
G16H 50/20(2018.01)i
FI: G16H50/20
According to International Patent Classification (IPC) or to both national classification and IPC

**B. FIELDS SEARCHED**

Minimum documentation searched (classification system followed by classification symbols)
G16H50/20

Documentation searched other than minimum documentation to the extent that such documents are included in the fields searched
Published examined utility model applications of Japan 1922–1996
Published unexamined utility model applications of Japan 1971–2021
Registered utility model specifications of Japan 1996–2021
Published registered utility model applications of Japan 1994–2021

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | JP 2005-202547 A (HITACHI, LTD.) 28 July 2005 (2005-07-28) paragraphs [0055]-[0057], [0062]-[0071], fig. 6 | 1, 8-9, 40, 47-48 |
| Y | paragraphs [0055]-[0057], [0062]-[0071], fig. 6 | 2-7, 10-11, 14-15, 18-22, 27-28, 33-39 |
| Y | paragraphs [0055]-[0057], [0062]-[0071], fig. 6 | 41-46, 49-50, 53-54, 57-61, 66-67, 72-78 |
| A | entire text, all drawings | 12-13, 16-17, 23-26, 29-32, 51-52, 55-56, 62-65, 68-71 |

☒ Further documents are listed in the continuation of Box C.   ☒ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| 29 March 2021 (29.03.2021) | 06 April 2021 (06.04.2021) |

| Name and mailing address of the ISA/ Japan Patent Office 3-4-3, Kasumigaseki, Chiyoda-ku, Tokyo 100-8915, Japan | Authorized officer Telephone No. |
|---|---|

Form PCT/ISA/210 (second sheet) (January 2015)

| International application No. |
| --- |
| PCT/JP2020/048146 |

C (Continuation).   DOCUMENTS CONSIDERED TO BE RELEVANT

| Category* | Citation of document, with indication, where appropriate, of the relevant passages | Relevant to claim No. |
| --- | --- | --- |
| Y | JP 2001-67403 A (CARE NETWORK KK) 16 March 2001 (2001-03-16) paragraphs [0010]-[0011], [0036]-[0037] | 2-7, 10-11, 14-15, 18-22, 27-28, 33-39 |
| Y | paragraphs [0010]-[0011], [0036]-[0037] | 41-46, 49-50, 53-54, 57-61, 66-67, 72-78 |
| A | entire text, all drawings | 12-13, 16-17, 23-26, 29-32, 51-52, 55-56, 62-65, 68-71 |
| Y | JP 2010-113668 A (TOSHIBA CORP.) 20 May 2010 (2010-05-20) paragraphs [0070]-[0073] | 10-11, 14-15, 18-22, 27-28, 33-39 |
| Y | paragraphs [0070]-[0073] | 49-50, 53-54, 57-61, 66-67, 72-78 |
| A | entire text, all drawings | 12-13, 16-17, 23-26, 29-32, 51-52, 55-56, 62-65, 68-71 |

Form PCT/ISA/210 (continuation of second sheet) (January 2015)

**INTERNATIONAL SEARCH REPORT**

Information on patent family members

International application No.

PCT/JP2020/048146

| Patent Documents referred in the Report | Publication Date | Patent Family | Publication Date |
|---|---|---|---|
| JP 2005-202547 A | 28 Jul. 2005 | (Family: none) | |
| JP 2001-67403 A | 16 Mar. 2001 | (Family: none) | |
| JP 2010-113668 A | 20 May 2010 | (Family: none) | |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 6203765 B
- JP 6217787 B
- JP 6288485 B
- JP 6342095 B
- JP 6566241 B
- JP 6577761 B